(19) Europäisches Patentamt
European Patent Office
Office européen des brevets

(11) Publication number : **0 343 133 B1**

(12) # EUROPEAN PATENT SPECIFICATION

(45) Date of publication of patent specification : **08.04.92 Bulletin 92/15**

(21) Application number : **89850146.5**

(22) Date of filing : **05.05.89**

(51) Int. Cl.$^5$ : **C07D 473/18,** C07D 473/32, C07D 473/34, A61K 31/52, A61K 31/675, C07F 9/547

(54) **Derivatives of purine, process for their preparation and a pharmaceutical preparation.**

The file contains technical information submitted after the application was filed and not included in this specification

(30) Priority : **06.05.88 SE 8801729**

(43) Date of publication of application : **23.11.89 Bulletin 89/47**

(45) Publication of the grant of the patent : **08.04.92 Bulletin 92/15**

(84) Designated Contracting States : **AT BE CH DE ES FR GB GR IT LI LU NL SE**

(56) References cited :
**EP-A- 0 108 285**
**EP-A- 0 146 516**
**EP-A- 0 186 640**
**EP-A- 0 242 482**
**GB-A- 2 134 907**
**ANTIMICROBIAL AGENTS AND CHEMOTHERAPY, Vol. 31, Jan. 1987, pages 76-80, G. ABELE et al.: "Ihibiting effect of (RS)-9-[4-hydroxy-2-hydroxymethyl)butyl]guanine on varicella-zoster virus replication in cell culture"**

(56) References cited :
**ANTIMICROBIAL AGENTS AND CHEMOTHERAPY, Vol. 30, No. 4, Oct. 1986, pages 598-605, ALF LARSSON et al.: "Mode of action, toxicity, pharmacokinetics, and efficacy of some new antiherpesvirus guanosine analogs related to buciclovir"**

(73) Proprietor : **Medivir Aktiebolag**
**Lunastigen 7**
**S-141 44 Huddinge (SE)**

(72) Inventor : **Lindborg, Björn Gunnar**
**Helgarögränd 14**
**S-125 42 Älvsjö (SE)**
Inventor : **Datema, Roelf**
**511 Cardinal Lane**
**Cheshire Connecticut 06410 (US)**
Inventor : **Johansson, Karl Nils Gunnar**
**Bäverstigen 19**
**S-150 23 Enhörna (SE)**
Inventor : **Öberg, Bo Fredrik**
**Askvägen 27**
**S-752 52 Uppsala (SE)**

(74) Representative : **Larfeldt, Helene et al**
**Bergenstråhle & Lindvall AB Sankt Paulsgatan 1**
**S-116 47 Stockholm (SE)**



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

Field of the invention

The present invention relates to novel and known chemical compounds and pharmaceutically acceptable salts thereof for use in therapy for therapeutic and prophylactic treatment of the acquired immuno deficiency syndrome (AIDS) and infections caused by viruses requiring reverse transcriptase for replication, such as human immunodeficiency viruses and hepatitis B viruses, and also for treatment of other virus diseases, such as those of herpes viruses, diseases which include both common infections and neoplastic diseases, i.e. cancer.

Background of the invention

The effects of viruses on bodily functions is the end result of changes occurring at the cellular and subcellular levels. The pathogenic changes at the cellular level are different for different combinations of viruses and host cells. While some viruses cause a general destruction (killing) of certain cells, other may transform cells into a neoplastic state.

Important common viral infections are herpes dermatitis (including herpes labialis), herpes keratitis, herpes genitalis, herpes zoster, herpes encephalitis, infectious mononucleosis and cytomegalovirus infections all of which are caused by viruses belonging to the herpes virus group. Other important viral diseases are influenza A and B which are caused by influenza A and B virus respectively. Another important common viral disease is viral hepatitis and especially hepatitis B virus infections are widely spread. Effective and selective antiviral agents are needed for treatment of these diseases as well as for other diseases caused by viruses.

Several different viruses of both DNA and RNA type have been shown to cause tumors in animals. The effect of cancerogenic chemicals can on animals result in activation of latent tumor viruses. It is possible that tumor viruses are involved in human tumors. The most likely human cases shown today are leukemias, sarcomas, breast carcinomas, Burkitt lymphomas, nasopharyngeal carcinomas and cervical cancers where RNA tumor viruses and herpes viruses are indicated. This makes the search for selective inhibitors of tumorogenic viruses and their functions an important undertaking in the efforts to treat cancer.

In the late seventies a new disease was reported, which subseqently was referred to as Acquired Immuno Deficiency Syndrome (AIDS). It is now generally accepted that a retrovirus referred to as HIV (Human Immunodeficiency Virus), formerly known as Human T-cell Lymphotropic Virus (HTLV-III) as Lymphadenopathy Associated Virus (LAV) plays an essential role in the etiology of AIDS. Different types of HIV have been found such as HIV-1 and HIV-2 and more are likely to be isolated.

AIDS is characterized by a profound immunodeficiency due to low numbers of a subset of lymphocyte-T-helper cells, which are one target for HIV-infection. The profound immunodeficiency in AIDS patients makes these patients highly susceptible to a variety of opportunistic infections of bacterial, fungal, protozoal or viral etiology. The etiological agents among viral opportunistic infections are often found in the herpes virus group, i.e. herpes simplex virus (HSV), Varicella Zoster virus (VZV), Epstein-Barr Virus (EBV) and, especially, cytomegalovirus (CMV). Other retroviruses affecting animals are feline leukemia virus and equine infectious anaemia virus. Human diseases such as multiple sclerosis, psoriasis and Kawasaki disease have also been reported to be associated with retrovirus infections.

Hepatitis B virus infections cause severe disease such as acute hepatitis, chronic hepatitis, fulminant hepatitis in a consider able number of persons. It is estimated that there are 200 million patients with chronic hepatitis B infection in the world. A considerable number of the chronic cases progress to liver cirrosis and liver tumours. In some cases the hepatitis infections are also take a rapid and severe course as in fulminant B hepatitis with about 90 % mortality. At present there is no known effective treatment against hepatitis B infections. The replication of hepatitis B virus is similar to that of retroviruses and it contains the same essential viral reverse transcriptase activity.

General outline of the invention

A great number of nucleoside analogues exhibit several antimetabolic activities. They do so by substituting for or competing with the naturally occuring nucleosides. Recently some nucleoside analogues have been described, which inhibit in cell culture the multiplication of human immunodeficiency virus (HIV, also called HTLV-III, LAV) the causative agent of AIDS and AIDS-related complex (ARC).

We have now found that activities for inhibition of HIV and/or herpes multiplication are exhibited by nucleoside analogues, in which the nucleoside bases are both natural and modified purine bases which in N-9 position are derivatized with an acyclic side chain, branched in the 2′-position, and containing functional groups.

Prior Art

Purine derivatives with antiviral activity have previously been disclosed in the following references: 9-(Phosphonylmethoxyalkyl)adenines are described in AU-A-56328/86 and AU-A-56468/86;
9-(1,3-dihydroxy-2-propoxymethyl)purines and cyclic phosphate esters are described in US-A-4,565,868, US-A-4-590,269 and EP-A-184 473; and
9-(4-hydroxy-3-hydroxymethylbutyl)purine derivatives are described in EP-A-141 927.

In addition the compound of the formula

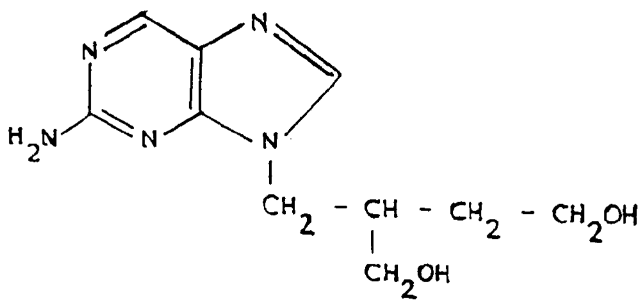

is known from EP-A-186 640;
and the compounds of the formula

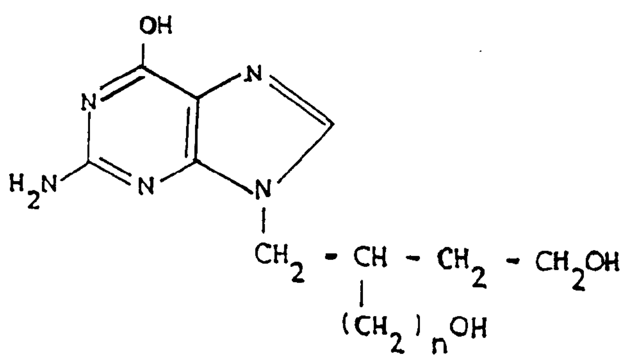

wherein n = 1 or 2, are known from EP-A-146 516.

Disclosure of the invention

It has been found according to the present invention that the compounds of the formula

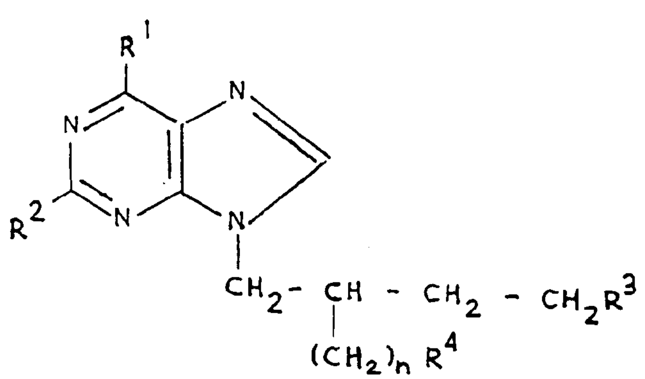

I

wherein:

$R^1$ is hydrogen, hydroxy, mercapto or amino;
$R^2$ is hydrogen, hydroxy, fluoro, chloro or amino;
$R^3$ and $R^4$ are independently selected from

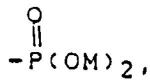,
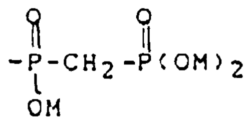,

amino, hydroxy
or an ether or ester residue thereof, or
$R^3$ together with $R^4$ is

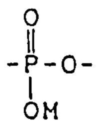,

wherein
M is hydrogen or a pharmaceutically acceptable counterion; and n is 1 or 2; and pharmaceutically acceptable salts thereof, inhibit the multiplication of human immunodeficiency virus (HIV). The compounds of the formula I are useful as therapeutic and/or prophylactic agents in the control and treatment of HIV virus infections in man.

In a more general aspect, the compounds of the formula I are useful as therapeutic and/or prophylactic agents in the control and treatment of infections caused by retroviruses and hepatitis B virus in mammals and man.

All retroviruses, including HIV, require the enzyme reverse transcriptase in their natural cycle of replication.

Hepatitis B virus (HBV) is a DNA virus with a unique circular double-stranded DNA genome which is partly single-stranded. It contains a specific DNA polymerase required for viral replication. This DNA polymerase also acts as a reverse transcriptase during the replication of HBV DNA via an RNA intermediate.

The compounds of the formula I inhibit the activity of reverse transcriptase of retroviruses including HIV as well as the activity of DNA polymerase of hepatitis B virus.

Another important area of use for the compounds of the formula I as in the treatment of herpes virus infections. Among the herpes viruses may be mentioned Herpes simplex type 1 and 2, varicella (Herpes Zoster), virus causing infections mononucleosis (i.e. Epstein-Barr virus) and cytomegalovirus. Important diseases caused by herpes viruses are herpes dermatitis (including herpes labialis), herpes genitalis, herpes keratitis, herpes encephalitis and herpes zoster.

Another possible area of use for the compounds of the present invention is in the treatment of cancer and tumors, particularly those caused by viruses. This effect may be obtained in different ways, i.e. by inhibiting the transformation of virus-infected cells to a neoplastic state, by inhibiting the spread of viruses from transformed cells to other normal cells and by arresting the growth of virus-transformed cells.

The present invention relates to the use of a compounds of the formula I

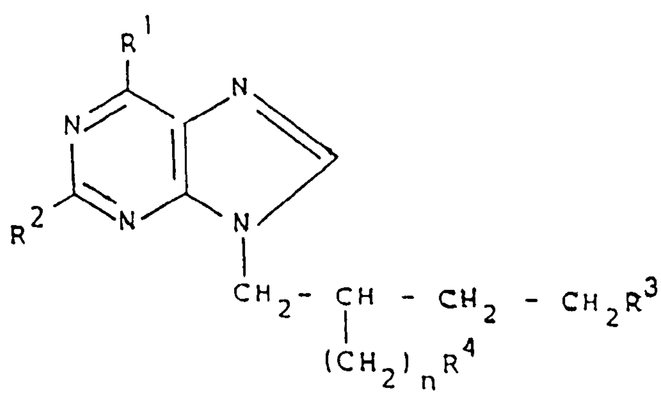

I

wherein:
$R^1$ is hydrogen, hydroxy, mercapto or amino;
$R^2$ is hydrogen, hydroxy, fluoro, chloro or amino;
$R^3$ and $R^4$ are independently selected from

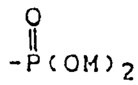, 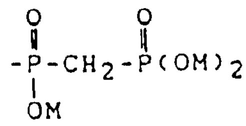,

amino, hydroxy
or an ether or ester residue thereof, or
$R^3$ together with $R^4$ is

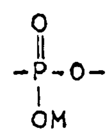 ,

wherein
M is hydrogen or a pharmaceutically acceptable counterion; and n is 1 or 2; and pharmaceutically acceptable salts thereof for the manufacture of a medicament for therapeutic and/or prophylactic treatment of the acquired immuno deficiency syndrome and infections caused by viruses requiring reverse transcriptase for replication.

Preferably they can be used for the treatment of infections caused by HIV viruses or hepatitis B virus.

The compounds of the formula I contain one asymmetric center when $CH_2CH_2R^3$ and $(CH_2)_nR^4$ are different. Accordingly they exist in two optical forms which constitute a further aspect of the invention.

Preferred compounds to be used in accordance with the invention are those wherein $R^1$ and $R^2$ are independently hydrogen, hydroxy or amino and wherein $R^3$ is

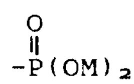,

hydroxy or an ester derivative thereof, and $R^4$ is OH or an ester derivative thereof or wherein $R^3$ and $R^4$ together are

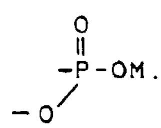

Preferably $R^3$ and $R^4$ are both hydroxy.

Examples of especially preferred compounds are those of the formula I

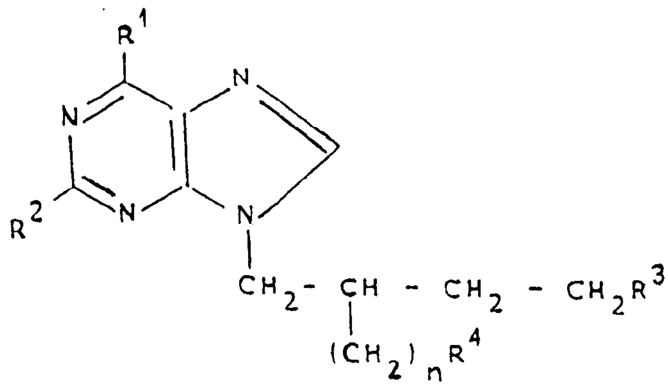

wherein

$R^1$ = OH, $R^2$ = $NH_2$, $R^3$ = OH, $R^4$ = OH
$R^1$ = H, $R^2$ = $NH_2$, $R^3$ = OH, $R^4$ = OH
$R^1$ = $NH_2$, $R^2$ = H, $R^3$ = OH, $R^4$ = OH

$R^1$ = OH, $R^2$ = $NH_2$, $R^3$ and $R^4$ = 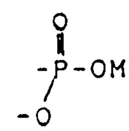

$R^1$ = H, $R^2$ = $NH_2$, $R^3$ and $R^4$ = 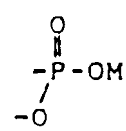

$R^1$ = $NH_2$, $R^2$ = H, $R^3$ and $R^4$ = 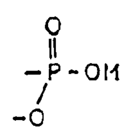

$R^1$ = OH, $R^2$ = $NH_2$, $R^3$ = $OCOC_{1-3}$, $R^4$ = $OCOC_{1-3}$
$R^1$ = H, $R^2$ = $NH_2$, $R^3$ = $OCOC_{1-3}$, $R^4$ = $OCOC_{1-3}$
$R^1$ = $NH_2$, $R^2$ = H, $R^3$ = $OCOC_{1-3}$, $R^4$ = $OCOC_{1-3}$
$R^1$ = OH, $R^2$ = $NH_2$, $R^3$ = OCONH-phenyl, $R^4$ = OCONH-phenyl
$R^1$ = H, $R^2$ = $NH_2$, $R^3$ = OCONH-phenyl, $R^4$ = OCONH-phenyl
$R^1$ = $NH_2$, $R^2$ = H, $R^3$ = OCONH-phenyl, $R^4$ = OCONH-phenyl

Esters and ethers of the purine derivatives are also included in the invention. Examples of esters are phosphate esters, carboxylic esters, carbonate esters, carbamate esters or sulphonic esters. The acid part of the esters may have alkyl, aryl or arylalkyl chains, where the aryl functionalities are optionally substituted for example by alkoxy, amino, nitrile, alkyl or sulphonamido groups or by one or more halogen atoms.

Examples of other types of derivatives of the purine bases are alkyl or arylalkyl derivatives of the primary hydroxyl group(s). The arylalkyl ether derivatives may be for example benzyl or triphenyl methyl and the aryl moiety may be optionally substituted. Furthermore, it is understood that the examples of the pharmaceutically acceptable salts cited below also apply to the various esters or derivatives of the purine bases of the invention.

In a compound of the formula I $R^3$ and $R^4$ as an ether residue can be defined as $OR^5$, wherein $R^5$ is $C_{1-6}$ alkyl, arylalkyl optionally substituted with one or more alkoxy, amino, nitrile or sulphamido groups or one or more

halogen atoms.

$R^3$ and $R^4$ as an ester residue can be derived from a carboxylic acid $R^6COOH$, a carbonic acid $R^7OCOOH$, a double ester of a carbonic acid $R^7CO_2CH(R^8)OCO_2H$, a sulphonic acid $R^7SO_2OH$, a carbamic acid $R^7NHCOOH$ or a phosphoric acid, wherein $R^6$ is hydrogen, $C_{1-17}$ alkyl, alkoxyalkyl, arylalkyl or aryl, $R^7$ is $C_{1-17}$ alkyl, arylalkyl or aryl, $R^8$ is hydrogen or $C_{1-3}$ alkyl and said aryl and arylalkyl groups optionally can be substituted with one or more alkyl, alkoxy, amino, nitrile, sulphonamide groups or one or more halogen atoms.

Examples of pharmaceutically acceptable salts of the compounds of formula I include base salts, e.g. derived from an appropriate base, such as alkali metal (e.g. sodium, potassium, alkaline earth metal (e.g. magnesium) salts, ammonium and $NX_4^+$ (wherein X is $C_{1-4}$ alkyl). Physiologically acceptable acid salts include salts of inorganic carboxylic acids such as acetic, lactic, gluconic, citric, tartaric, maleic, malic, pantothenic, isethionic, oxalic, lactobionic and succinic acids; organic sulfonic acids such as methanesulfonic, ethanesulfonic, benzenesulfonic, p-chlorobenzenesulphonic and p-toluenesulfonic acids and inorganic acids such as hydrochloric, hydroiodic, sulfuric, phosphoric and sulfamic acids.

Physiologically acceptable counterions of the phosphonate groups include inorganic and organic counterions. Inorganic counterions are for example ammonium, sodium, potassium, lithium, magnesium and calcium. Organic counterions are derived from non-toxic bases, such as primary, secondary and tertiary amines, including naturally occuring amines. Examples of such amines are diethylamine, triethylamine, isopropylamine, ethanolamine, morpholine, 2-diethylaminoethanol, glucosamine, N-methylglucamine, piperazine and dicyclohexylamine.

The present invention also relates to novel compounds of the formula

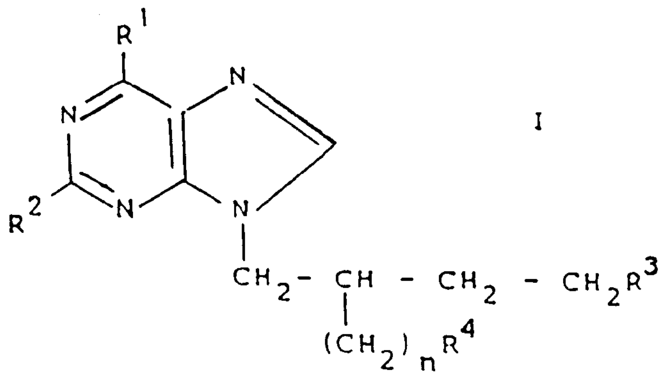

I

wherein:
$R^1$ is hydrogen, hydroxy, mercapto or amino;
$R^2$ is hydrogen, hydroxy, fluoro, chloro or amino;
$R^3$ and $R^4$ are independently selected from

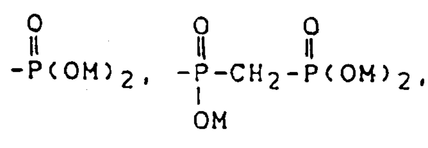

amino, hydroxy
or an ether or ester residue thereof, or
$R^3$ together with $R^4$ is

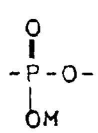

,

wherein
M is hydrogen or a pharmaceutically acceptable counterion; and n is 1 or 2; with the proviso that, when $R^2$ is

amino and $R^3$ and $R^4$ are hydroxy, $R^1$ is not hydroxy and in addition, when n = 1, $R^1$ is not hydrogen, and pharmaceutically acceptable salts thereof.

The invention furthermore provides:

A pharmaceutical composition comprising a new compound of the formula I as active ingredient; and

A method for therapeutic and/or prophylactic treatment of virus infections in an aminal or human host in need of treatment comprising administering an effective amount of a new compound of the formula I.

It is a preferred aspect of the invention to treat infections caused by herpes virus or a virus requiring reverse transcriptase for replication, including human immuno deficiency viruses and hepatitis B virus.

In clinical practice the purine derivatives of the formula I will normally be administered orally, by injection or by infusion in the form of a pharmaceutical preparation comprising the active ingredient in the form of the original compound or optionally in the form of a pharmaceutically acceptable carrier which may be a solid, semi-sold or liquid diluent or an ingestible capsule. The compound may also be used without carrier material. As examples of pharmaceutical preparations may be mentioned tablets, dragées, capsules, granulates, suspensions, elixirs, syrups, solutions etc. Usually the active substance will comprise between 0.05 and 20 % for preparations intended for injection and between 10 and 90 % for preparations intended for oral administration.

In the treatment of patients suffering from retrovirus, especially HIV, or hepatitis B virus infections, it will be preferred to administer the compounds by any suitable route including the oral, parenteral, rectal, nasal, topical and vaginal route. The parenteral route includes subcutaneous, intramuscular and intravenous administration. The topical route includes buccal and sublingual administration. The dosage at which the active ingredients are administered may vary within a wide range and will depend on various factors such as the severity of the infection, the age of patient etc., and may have to be individually adjusted. As a possible range for the amount of the compounds of the invention or a physiologically acceptable salt thereof to be administered per day may be mentioned from about 10 mg to about 10 000 mg, preferentially 100-500 mg for intravenous administration and preferentially 100-3000 mg for oral administration.

Compounds of the formula I can cooperate synergistically or additively with a wide range of other therapeutic agents, thereby enhancing the therapeutic potential of both agents without adding the toxic effects, thus increasing the therapeutic ratio.

Therefore, a compound of formula I or a pharmaceutically acceptable derivative thereof can be used in combination therapy, wherein the two active agents are present in a ratio resulting in an optimal therapeutic ratio. This can be provided either by a synergistic effect against the viral infection and/or by a decrease in toxicity while maintaining a therapeutic effect which is additive or synergistic.

The optimal therapeutic ratio is observed when the two agents are present in a ratio of 500:1 to 1:500, preferably 100:1 to 1:100, particularly 20:1 to 1:20 and especially 10:1 to 1:10.

Said combinations may conveniently be administered together, for example, in a unitary pharmaceutical formulation, or separately for example as a combination of tablets and injections administered at the same time or different times, in order to achieve the required therapeutic effect.

The compounds of the formula I are potentiated by interferons, other antiviral agents such as foscarnet, AZT, HIV protease inhibitors, immunomodulators, interferon inducers and growth factors.

Particularly preferred types of interferon are $\alpha$, $\beta$ and $\gamma$ and interferon inducers such as "Ampligen" (Hem Research).

Other combinations suitable for use according to the present invention include those wherein the second agent is, for example, interleukin II, suramin, foscarnet or an ester thereof, HPA 23, inhibitors of HIV protease such as pepstatin, steroids, medications such as levamisol or thymosin to increase lymphocyte numbers and/or function as appropriate, or GM-CSF and other factors regulating cell functions.

## Methods of preparation

The compounds of the invention may be prepared by one of the following general methods, constituting a further aspect of the invention.

A. Condensing an acyclic side chain as comprised in formula I, to the N-9 position of a purine derivative. The acyclic side chain has a terminal leaving group and the functional groups may be optionally protected with known groups used for protection of hydroxy, amino or phosphonate functions.

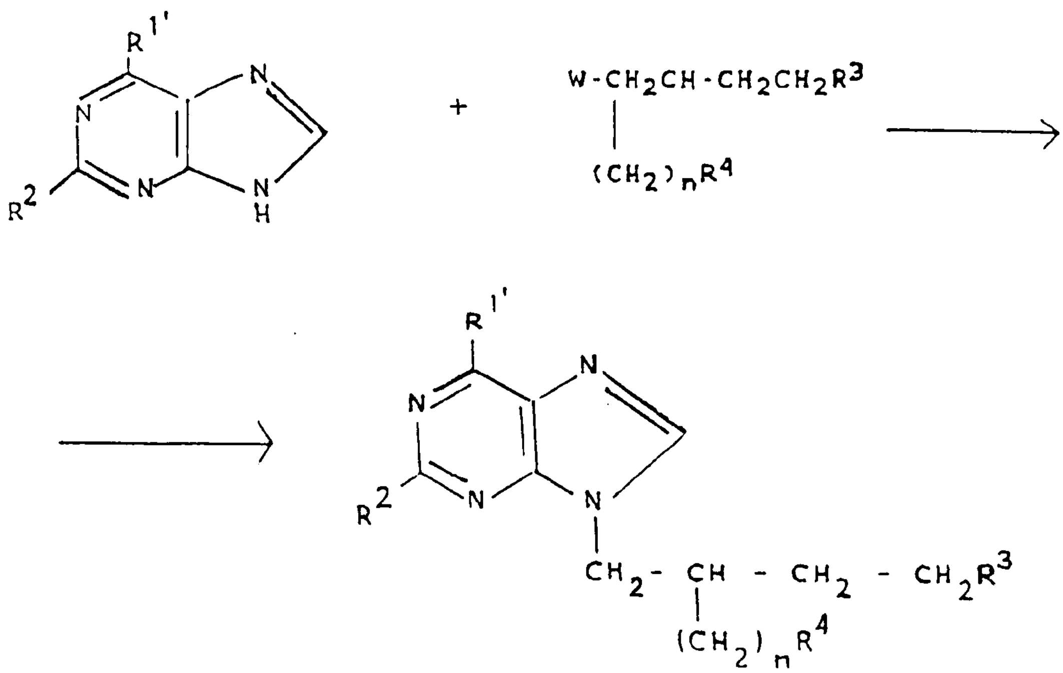

Examples of suitable derivatives of the reacting species are those wherein R1' is Cl, or $R^1$ as defined above, $R^1$, $R^2$, $R^3$, $R^4$ and n are as defined above, and W is a suitable leaving groups, such as Cl, Br, J, alkyl or aryl sulfonyloxy, trifluoromethanesulfonyloxy. The condensation reaction is performed in an organic soluent such as dimethyl formamide, dimethylsulfoxyde, ethanol, acetonitrile, dichloromethane or the like at a temperature of between 0°C and 150°C for 1 hour to 5 days, and after condensation the products may be hydrolyzed or converted by conventional methods, known to those skilled in the art, into compounds of the formula I.

For the case of a phosphonate the side chains condensed to a purine base could be prepared in different ways. One example is the following reaction sequence, where the starting material 5-(2-bromoethyl)-2,2-dimethyl-1,3-dioxane has been described (M.R. Harnden and R.L. Jarvest, Tetrahedron Letters, Vol. 26, pages 4265-4268, 1985).

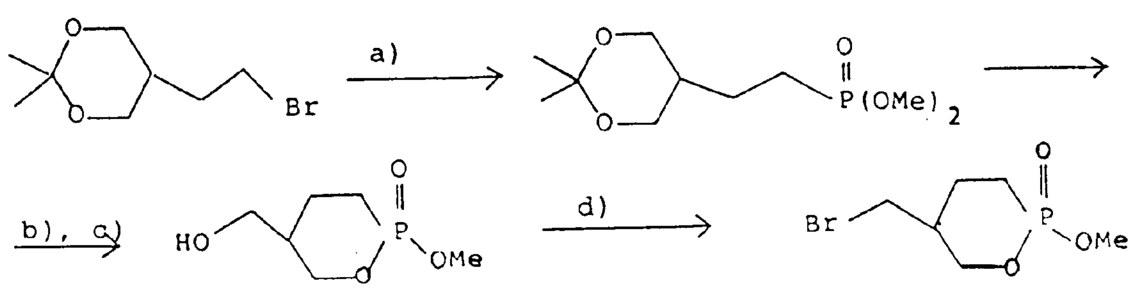


a) $P(OMe)_3$; B) $H^+$, MeOH; c) MeO-; d) N-bromosuccinimide, triphenylphosphine;

B. Imidazole ring closure of a substituted pyrimidine derivative to the purine base followed by removal of the protecting groups.

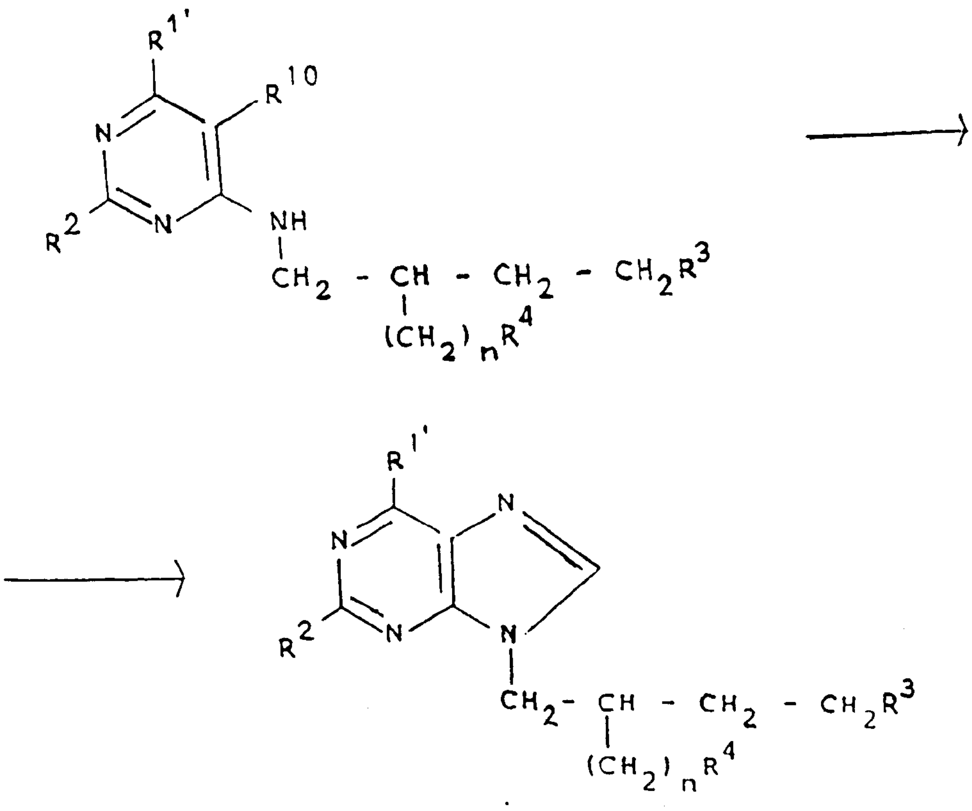

$R^1$, $R^2$, $R^3$ and $R^4$ are as defined above, $R^{10}$ is nitroso, nitro, amino or an amino derivative such as formylamino or orthoesteramino. The ring closure may be performed by known methods (the principles which are given for example by E. Lunt in Comprehensive Organic Chemistry (Eds. D. Barton and W.B. Ollis, Pergamon Press 1979) vol 4, p. 499-505 and by G. Shaw in Comprehensinve Heterocyclic Chemistry (Eds. A.R. Katritzsky and C.W. Reese, Pergamon Press 1984) vol. 5, p. 570-573. The reaction may be performed in an organic solvent such as for example, formic acid, formamide, orthoformate ester or diethoxymethylacetate at a temperature from 25°C to 250°C for 10 minutes to 24 hours. When $R^{10}$ is nitroso or nitro, these groups first have to be reduced to an amino group by any known method.

C. Imidazole ring closure via a furazano[3,4-d]pyrimidine ring system to the purine base followed by removal of the protecting groups.

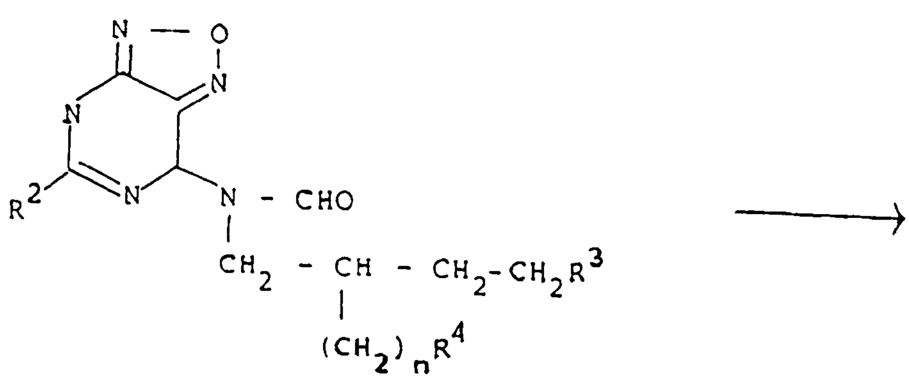

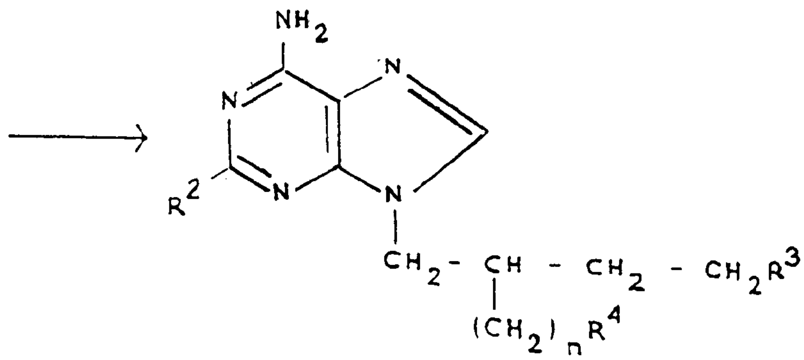

$R^2$, $R^3$ and $R^4$ are as defined above. The ring closure may be performed by heating following reductive cleavage of the furazane ring by for example zink in acetic acid. After reaction the 6-$NH_2$ group of the purine may optionally be transformed to a hydroxy group by treatment with for example sodium nitrile in acetic acid.

D. Pyrimidine ring closure to the purine base followed by removal of the protecting groups.

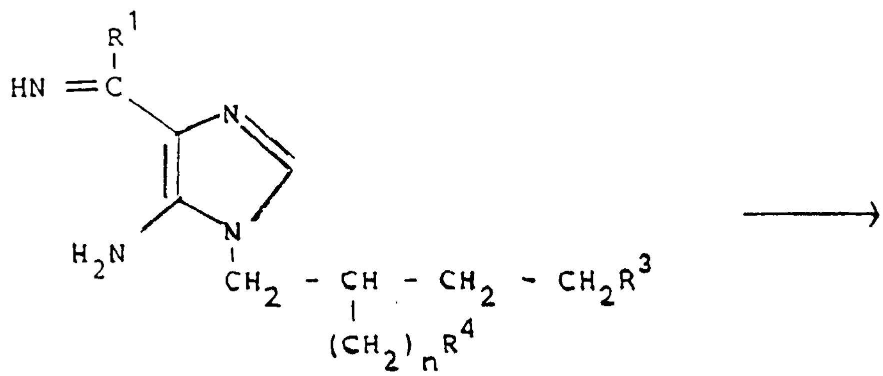

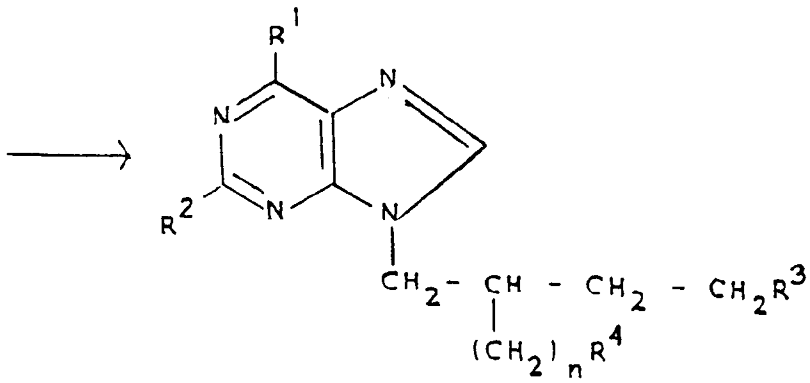

The ring closure may be performed according to known methods which have been described for example by G. Shaw in Comprehensive Heterocyclic Chemistry (Eds. A.R. Katritzsky and C.W. Reese, Pergamon Press 1984) Vol. 5, p. 583-591 and by E. Lunt in Comprehensive Organic Chemistry (Eds. D. Barton and W.B. Ollis, Pergamon Press 1979) Vol. 4, p. 505-508.

The described methods A-D may be used to give mixtures of optical isomers, or in appropriate cases a single optical isomer. A compound according to the invention in the form of an optical isomer can be prepared if in method A either an optically active acyclic side chain is condensed to the N-9 position of the purine derivative or the condensation is directed to the formation of an optical isomer by means of another optically active compound, and in methods B-D starting materials having an optically active side chain are subjected to the ring closure. Additionally a single optical isomer may be obtained from the racemic mixtures by methods known per se.

The following examples will further illustrate the invention.

Example 1 2-(2-Aminopurin-9-yl)methylbutane-1,4-diol

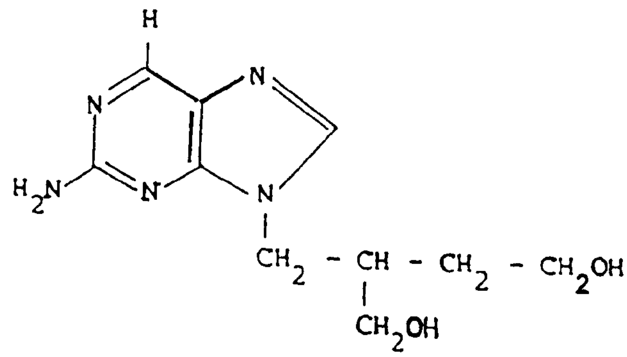

VSB 212

To a solution of crude dimethyl (2-aminopurin-9-ylmethyl)succinate (3.2 g, 10.9 mmol), dissolved in tert. butanol (250 ml) at 40°C, was added lithium borohydride (1.3 g, 60 mmol) in portions with stirring. After 1 hour at ambient temperature, water (30 ml) was added slowly and stirring continued over night. Inorganic salts were filtered and the solution evaporated to dryness. Yield of crude product was 1.6 g (50 %). Chromatography on silica (chloroform + methanol 7+1) afforded pure product.

$^{1}H$ NMR (DMSO-$d_6$) : δ 31.4 (m, 2H) $CH_2CH_2OH$; 2.14 (m, 1H) CH; 3.33 (d, 2H) CH-$CH_2OH$; 3.44 (diffuse q, 2H); 4.05 (AB part of ABX, 2H) N-$CH_2$; 6.37 (broad s, 2H) $NH_2$; 7.99 (s, 1H) H8; 8.56 (s, 1H) H6.

$^{13}C$ NMR ($D_2O$): δ 33.11 $CH_2CH_2OH$; 39.58 CH; 46.34 $NCH_2$; 61.35 and 63.51 $2xCH_2OH$; 128.41 C5; 146.92 C8; 150.48 C6; 155.05 C4; 161.50 C2.

The starting material dimethyl 2-(2-aminopurin-9-ylmethyl)succinate was prepared as follows (a,b):

a) Dimethyl 2-(2-amino-6-chloropurin-9-ylmethyl)succinate

A mixture of 2-amino-6-chloropurine (4.07 g, 0.024 mol), dimethyl itaconate (5.00 g, 0.032 mol), and sodium hydride (55 % in oil, 0.2 g) in 50 ml of dry dimethylformamide was stirred at room temperature for 3 days. About 50 ml of water was added and the mixture was washed with n-hexane (2x50 ml) and then extracted with 2x50 ml of dichloromethane. The combined $CH_2Cl_2$ extracts were washed with 2x20 ml of water, dried with magnesium sulfate, and evaporated in vacuum. Treatment with ether and drying afforded a white crystalline product. Chromatograpy (silica gel, chloroform + methanol 15+1) yielded 5.54 g (71 %) or recrystallization (MeOH-$H_2O$) yielded 5.15 g (66.1 %) of dimethyl 2-(2-amino-6-chloropurin-9-ylmethyl)succinate.

UV spectrum in EtOH, λ max (nm): 310 (247).

$^{1}$NMR ($CDCl_3$) δ 2.67 (dd, 2H) $CH_2COO$; 3.46 (m, 1H) CH; 3.70 (2s, 2x3H) $OCH_3$; 4.42 (ABX system, Jgem = 14 Hz, 2H) $NCH_2$; 5.35 (broad s, 2H) $NH_2$; 7.79 (s, 1H) H8.

b) Dimethyl 2-(2-aminopurin-9-ylmethyl)succinate

A mixture of dimethyl 2-(2-amino-6-chloropurin-9-ylmethyl)succinate (3.28 g, 10 mmol), sodium acetate (1.5 g) and 5 % palladium on charcoal (0.4 g) in ethanol (200 ml) was hydrogenated with agitation in a Parr apparatus at 40 psi for 115 h/room temperature. After filtration, sodium acetate (1.6 g) and 5 % Pd/C (0.4 g) were added and the hydrogenation was continued for 70 h. After filtration and evaporation to dryness, the residue was extracted with 2x50 ml of chloroform and the combined extracts were evaporated to dryness affording 2.6 g (89 %) of crude dechlorinated product.

$^{1}H$ NMR (DMSO-$d_6$) δ 8.00 (s, 1H) H8; 8.56 (s, 1H) H6.

Example 2 2-(2-Aminopurin-9-yl)methylbutane-1,4-diol diacetate

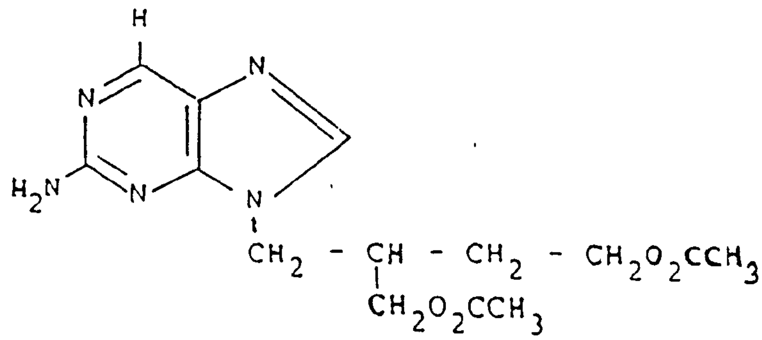

VSC 610

A mixture of 4-acetoxy-2-bromomethylbutyl acetate (0.465 g, 1.74 mmol), 2-aminopurine (0.282 g, 2.09 mmol), and powdered potassium carbonate (1.20 g, 8.70 mmol) in N,N-dimethylformamide (20 ml) was stirred at room temperature for 5 days. Chloroform (40 ml) was added, solid material was removed by filtration, and the solution was evaporated in vacuum to small volume. Chromatography on 50 g $SiO_2$ with chloroform + methanol (7+1) as eluent gave a fraction 70-130 ml, which was evaporated and dried in vacuum, finally at 0.1 mBar to give 0.349 g (62 %) of 2-(2-aminopurin-9-yl)methylbutane-1,4-diol diacetate. TLC on silica (chloroform + methanol 7+1): $R_f$ 0.57.

$^1$H NMR ($CDCl_3$+$CD_3OD$); δ 8.64 s, 1H) H6; 7.92 (s, 1H) H8; 5.82 (broad s, 2H) $NH_2$; 4.3-4.15 (m, 4H) 2 $CH_2OAc$; 4.33 (d, 2H) $CH_2N$; 2.50 (m, 1H) CH; 2.06 (s, 6H) 2 $CH_3COO$; 1.75 (q, 2H) $\underline{CH_2}CH_2OAc$; $^{13}$C NMR ($CDCl_3$+$CD_3CD$): δ 170.96, 170.66 (2 C=O); 159.89 (C2); 153.13 (C4); 148.77 (C6); 142.84 (C8); 126.83 (C5) 63.78 ($CHCH_2$-OAc); 61.47 ($CH_2\underline{C}H_2OAc$); 44.05 ($CH_2N$); 35.15 (CH); 27.59 ($\underline{C}H_2CH_2O$); 20.22, 20.05 (2 $CH_3$).

The starting materials were prepared by the following sequence of reactions (a-e):

a) α-Trityloxymethyl-γ-butyrolactone

A mixture of α-hydroxymethyl-γ-butyrolactone (26.83 g, 0231 mol) (G. Claeson and H.-G. Jonsson, Arkiv för Kemi 28, 167 (1967)), trityl chloride (77.3 g, 0.277 mol) and dry pyridine (200 ml) was stirred at room temperature for a few hours until homogeneous. After 10 days at room temperature the solution was poured into a mixture of 500 ml water and 500 ml n-hexane. The precipitate was washed with water and hexane and dried finally at 0.1 mBar to give 65.60 g (79 %) of crude product, contaminated with some trityl alcohol. TLC on silica (ethyl acetate + n-hexane 1+3): $R_f$ 0.38.

$^{13}$C NMR ($CDCl_3$): δ 177.84 (C=0); 143.71, 128.68, 127.96 and 127.20 (phenyl); 86.96 (O $\underline{C}PH_3$); 67.26 ($\underline{C}H_2OCO$); 62.49 ($\underline{C}H_2OTr$); 40.31 (CH); 26.15 ($\underline{C}H_2CH_2O$).

b) 2-Trityloxymethyl-1,4-butanediol

α-Trityloxymetyl-γ-butyrolactone (60.21 g, 0.168 mol) was added in small portions to a stirred suspension of lithium aluminium hydride (9.53 g, 0.251 mol) in dry tetrahydrofuran (300 ml) and the mixture was refluxed for 1 h. Slow addition of 10 ml $H_2O$ + 10 ml 15 % NaOH and 30 ml $H_2O$ produced a white sandy precipitation which was filtered off and washed with 2x50 ml tetrahydrofuran. The filtrate was evaporated to a small volume and dissolved in diethyl ether (300 ml), silica gel (250 g) was added and the mixture was carefully evaporated to a homogeneous powder. In a chromatography column the crude product - silica gel mixture was placed on top of silica gel (250 g) in n-hexane. Eluting with ethyl acetate + n-hexane (1+3), 2200 ml, removed trityl alcohol. Further eluting with ethyl acetate + ethanol (9+1) gave fractions 2900-3700 ml (from start), which after evaporation in vacuum produced a crystallizing oil. Yield 52.77 g (87 %). TLC on silica: ethyl acetate + n-hexane (1+3), $R_f$ 0.05; ethyl acetate + ethanol (9+1), $R_f$ 0.81.

c) 2-Trityloxymethyl-1,4-butanediol diacetate

To a stirred mixture of 2-trityloxymethyl-1,4-butandiol (50.85 g, 0.140 mol) and triethylamine (42.6, 0.42 mol) in dry diethyl ether (500 ml) was added slowly a solution of acetyl chloride (27.5 g, 0.35 mol) in ether (25 ml) with external cooling with cold water to maintain room temperature in the mixture. After 45 min the triethylamine hydrochloride was filtered off and washed with a little ether. The combined filtrate was washed with water (50 ml), 0.5 M hydrochloric acid (100 ml) and water (50 ml), dried with magnesium sulfate and evaporated in vacuum, finally at 0.1 mBar, to give 61.03 g (97 %) of crude oily product. TLC on silica (ethyl acetate + n-hexane 1+1): $R_f$ 0.68.

d) 4-Acetoxy-2-hydroxymethylbutyl acetate

2-Trityloxymethyl-1,4-butanediol diacetate (60.90 g, 0.136 mol) was dissolved in acetic acid (320 ml) at 100°C and water, (80 ml) was added. The solution was kept at 100°C for 15 min, evaporated in vacuum to small volume and cooled to 0°C. The precipitate was filtered off and washed with cold ethyl acetate to give 26.44 g (theory 35.51 g) of tritylalcohol. The combined filtrate was evaporated to small volume. The compound was purified on a silica gel column (500 g $SiO_2$); eluent 0-2700 ml ethyl acetate + n-hexane (1+1), 2700-3740 ml ethyl acetate + n-hexane (2+1), then neat ethyl acetate. The fractions 2540-4800 ml were evaporated to give 14.20 g (51 %) of pure 4-acetoxy-2-hydroxy-methylbutyl acetate. TLC on silica (ethyl acetate + n-hexane 1+1): $R_f$ 0.30.
$^{13}C$ NMR ($CDCl_3$): δ 171.08, 170.88 (2 C=O); 64.07 ($CH_2OH$); 62.10, 61.45 (2 $CH_2OAc$); 37.07 (CH); 26.76 ($CH_2CH_2OAc$); 20.39 (2 $CH_3$).

e) 4-Acetoxy-2-bromomethylbutyl acetate

A solution of 4-acetoxy-2-hydroxymethylbutyl acetate (11.04 g, 0.054 mol) and triphenylphospine (21.27 g, 0.081 mol) in dry dichloro methane (150 ml) was stirred at 0°C, and N-bromo-succinimide (14.43 g, 0.081 mol) was added in portions. The mixture was kept at 0°C for 20 h, evaporated to small volume and stirred with 50 ml of ethyl acetate + n-hexane (1+1). The white triphenylphosphine oxide was filtered off and washed with a little ethyl acetate + n-hexane (1-1). The combined filtrate was evaporated and purified on a 200 g $SiO_2$ column with ethyl acetate + n-hexane (1+1) as eluent. The 250-550 ml fraction was evaporated in vacuum to give 11.90 g (82 %) of pure 4-acetoxy-2-bromomethylbutyl acetate. TLC on silica (ethyl acetate + n-hexane 1+1): $R_f$ 0.59.
$^{1}H$ NMR ($CDCl_3$): δ 4.2-4.0 (m, 4H) 2 $CH_2OAc$; 3.53 (ABX system, 2H) $CH_2Br$; 2.25-2.1 (m, 1H) CH; 2.08, 2.06 (2 s, 2x3H) 2 $COCH_3$; 1.79 (m, 2H) $CH_2CH_2OAc$.
$^{13}C$ NMR ($CDCl_3$): δ 170.91, 170.74 (2 C=O); 64.90 ($CHCH_2OAc$); 61.71 ($CH_2CH_2OAc$); 36.56 (CH); 34.74 ($CH_2Br$); 28.88 ($CH_2CH_2O$); 20.95 ($2CH_3$).

Example 3 9-(4-Acetoxy-2-acetoxymethylbutyl)guanine [2-(guanin-9-ylmethyl)-1,4-butanediol diacetate]

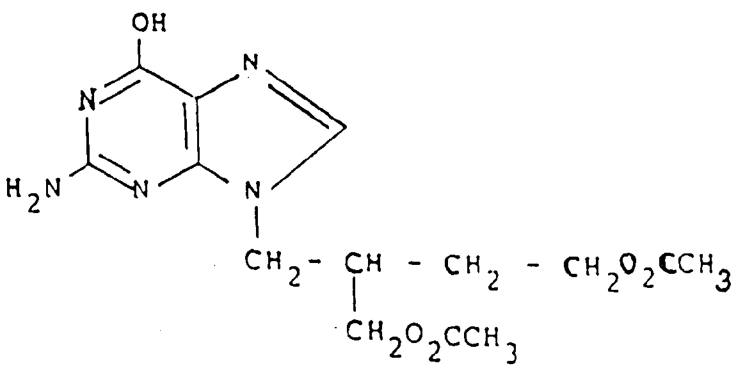

VSA 639

A mixture of 9-(4-hydroxy-2-hydroxymethylbutyl)guanine (0.50 g, 2.0 mmol), acetic anhydride (1.02 g, 10.0 mmol), pyridine (1.11 g, 14.0 mmol), and dry N,N-dimethylformamide (25 ml) was stirred at room temperature for 13 days and then evaporated to dryness in vacuum. The crystalline residue was heated with 10 ml of water and lyophilized and recrystallized from water to give 0.468 g (69 %) of 9-(4-acetoxy-2-acetoxymethylbutyl)guanine.
$^{13}C$ NMR ($CDCl_3$+$CD_3OD$): δ 64.15 ($CHCH_2O$); 61.98 ($CH_2CH_2O$); 44.71 ($CH_2N$); 35.88 (CH); 27.95 ($CH_2CH_2O$); 20.87, 20.68 (2 $CH_3$).

Example 4 9-(4 Propionoxy-2-propionoxymethylbutyl)guanine [2-(guanin-9-ylmethyl)-1,4-butanediol dipropionate]

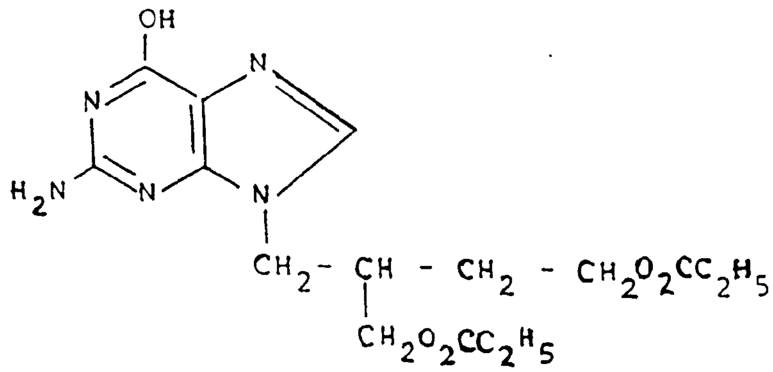

VSC 637

A mixture of 9-(4-hydroxy-2-hydroxymethylbutyl)guanine (0.50 g, 2.0 mmol), propionic anhydride (1.56 g, 12.0 mmol), pyridine (1.27 g, 16.0 mmol), and dry N,N-dimethylformamide (25 ml) was stirred at room temperature for 14 days and then evaporated to dryness in vacuum. The crystalline residue was heated with 10 ml of water and lyophilized and recrystallized from water to give 0.418 g (57 %) of 9-(4-propionoxy-2-propionoxymethylbutyl)-guanine.

$^{13}C$ NMR ($CDCl_3$+$CD_3OD$): δ 64.00 ($CH\underline{C}H_2O$); 61.86 $CH_2\underline{C}H_2O$); 44.78 ($CH_2N$); 35.95 (CH); 28.00 ($\underline{C}H_2CH_2O$); 27.56, 27.44 (2 $CH_3\underline{C}H_2CO$); 9.00 (2 $CH_3$).

Example 5 (-)-9-(4-Hydroxy-2-hydroxymethylbutyl)guanine

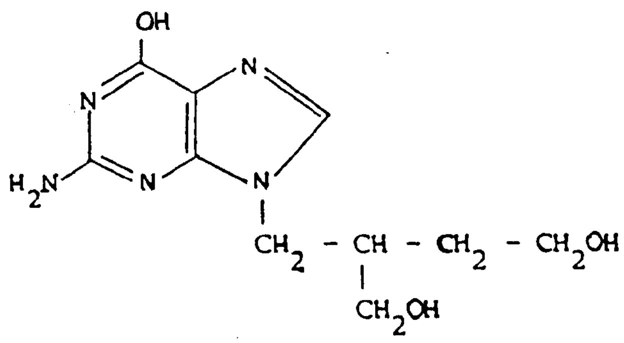

VSB 647

A solution of (-)-2-(2-amino-6-chlorpurin-9-ylmethyl)-1,4-butanediol (11.5 mg, 0.0423 mmol) in 50 % aqueous formic acid (0.75 ml) was kept at 100°C/2 h and then evaporated to dryness, dissolved in 2 ml of water and lyophilized. The product was dissolved in 1 ml of water, 2 drops of conc. aqueous ammonia was added and the solution kept at 100°C for 10 min, flushed with nitrogen to remove ammonia, and lyophilized. The residue was dissolved by warming with 1.2 ml of 20 % aqueous methanol and the solution filtered and kept in open air to allow for slow partial evaporization of solvent. Crystalline needles were formed. Filtration, washing with 3 drops of water and drying yielded 6.4 mg (60 %) of (-)-9-(4-hydroxy-2-hydroxymethylbutyl)guanine.

The compound was found to be levorotatory (ethanol, 589 and 546 nm). It produced a proton NMR (DMSO-$d_6$) identical to that of the racemate. TLC on silica (ethyl acetate + methanol + water 7+2+1): $R_f$ 0.37, identical to that of the racemate.

The starting material was prepared as follows (a-b):

a) (-)-Dimethyl-2-(2-amino-6-chloropurin-9-ylmethyl)succinate

The racemic compound was resolved by repeated chromatography on a microcrystalline triacetylcellulose column, (Perstorp Biochem, Lund, Sweden) with 95 % ethanol as mobile phase. The slower moving (-)-enantiomer produced a proton NMR spectrum identical to that of the racemic compound (±). The resolution was followed by proton NMR in deuterochloroform at 200 MHz with tris-[3-(heptafluoropropylhydroxymethylene)-d-camphorato]-europium(III) as chiral shift reagent. By addition of 1-1.5 parts (per weight) of shift reagent, the methyl ester signal of the racemate (2 close singlets at 3.69 and

3.695 ppm) were split into one base-line separated low-field pair (low-field signal from the (+)-enantiomer high-field signal from the (-)-enantiomer) and one less resolved high-field pair. The enantiomeric excess was then calculated from the ratio of the low-field signals.

b) (-)-2-(2-Amino-6-chloropurin-9-ylmethyl)-1,4-butanediol

To a solution of (-)-dimethyl-2-(2-amino-6-chloropurin-9-ylmethyl)succinate (enantiomeric excess 85 %; 19.9 mg, 0.0607 mmol), dissolved in tert. butanol (2.0 ml) at 40°C, was added lithium borohydride (30 mg, 1.38 mmol) in portions with stirring. After 1 h at ambient temperature, water (0.3 ml) was added slowly and stirring continued over night. Inorganic salts were filtered, washed carefully with tert. butanol and the solution was evaporated to dryness. Preparative thin-layer chromatography (PSC-Fertigplatten, Merck) with chloroform + methanol (5+1) as mobile phase afforded 16.5 mg (theoretical yield) of (-)-2-(2-amino-6-chloropurin-9-ylmethyl)-1,4-butanediol. $[\alpha]_D^{20°}$ - 5.20°, $[\alpha]_{546}^{20}$ - 5.92° (c 0.625, ethanol). TLC on silica (chloroform + methanol 5+1): $R_f$ 0.40, identical to that of the racemate.

Example 6 9-(4-Hydroxy-2-hydroxymethylbutyl)adenine

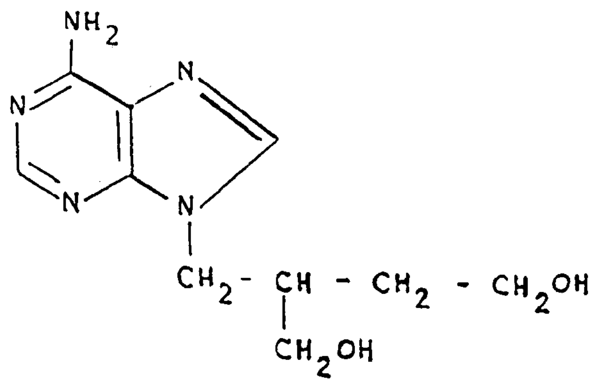

VSC 600

Dimethyl 2-(adenine-9-ylmethyl)succinate (2.93 g, 0.010 mol) was dissolved by warming with tert. butanol (120 ml), lithium borohydride (1.10 g, 0.05 mol) was added in portions and the mixture was stirred at ambient temperature for 3 h, water (10 ml) was added and stirring was continued over night. Inorganic material was filtered off and washed with tert. butanol and the filtrate evaporated to small volume. Chromatography on silica (ethyl acetate + methanol + water 7+2+1) afforded pure 9-(4-hydroxy-2-hydroxymethylbutyl) adenine.
$^{13}C$ NMR (DMSO-$d_6$): δ 156.24 (C6); 152.67 (C2); 150.14 (C4); 141.84 (C8); 118.88 (C5); 61.18, 58.92 (2 $CH_2OH$); 44.81 ($CH_2N$); 38.36 (CH); 32.02 ($\underline{C}H_2CH_2OH$).
The starting material was prepared as follows:

Dimethyl 2-(adenin-9-ylmethyl)succinate

A mixture of adenine (5.40 g, 0.040 mol), dimethyl itaconate (8.00 g, 0.051 mol), sodium hydride (55 % in oil, 0.2 g) and dry N,N-dimethylformamide (125 ml) was warmed to 120°C and then kept with stirring at room temperature for 8 days. The precipitate was filtered, washed with dichloromethane (3x15 ml) and dried in vacuum to yield 8.96 g (76 %) of dimethyl 2-(adenin-9-ylmethyl)-succinate.
1H NMR ($CDCl_3$); δ 8.28 (s, 1H) H2; 7.90 (s, 1H) H8; 4.54 (ABX system 2H) $CH_2N$; 3.70, 3.69 (2 s, 2x3H) $OCH_3$; 3.46 (m, 1H) CH; 2.72 (d, 2H) $CH_2COO$.
13C NMR ($CDCl_3$+$CD_3OD$): δ 172.39, 171.42 (2 C=O); 155.61 (C6); 152.91 (C2); 149.87 (C4); 141.01 (C8); 118.85 (C5); 52.37, 51.94 (OCH3); 44.10 ($CH_2N$); 41.55 (CH); 33.30 ($\underline{C}H_2COO$).

Example 7 Sodium ethyl 3-(guanin-9-ylmethyl)-4-hydroxybutanephosphonate and 7 isomer

VSC 658

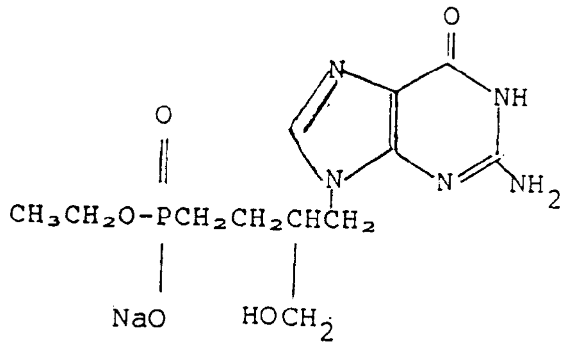

2-Amino-6-chloro-9-[(2-ethoxy-2-oxo-1,2-oxaphosphorinan-5-yl)-methyl]purine (VSC 655) and its 7 isomer (100 mg, 0.29 mmol), dissolved in ethanol (4 ml), water (4 ml), and 2M aqueous sodium hydroxide (0.90 mmol) was kept at 37°C for 18 h. The solution was neutralized by addition of weakly acidic Amberlite cation exchange resin, filtered, and evaporated to dryness to give 113 g (quantitative yield) of a crude product.
'H NMR ($D_2O$, tert BuOH, 200 Mhz); δ 8.01 and 7.79 (s, 8H, 7 and 9 isomers); ~ 4.03 (m, $CH_2N$); 3.78 (quintet, $CH_2OP$); 3.50 (d, $CH_2OH$); 2.05 and 1.6-1.3 (m, $PCH_2CH_2CH$); 1.12 (t, $CH_3C$-O-P).
$^{13}C$ NMR ($D_2O$, tert. BuOH, 50 MHz): δ 161.81, 160.47, 154.12, 145.5, 142.13, 114.55, 61.74/61.42 ($CH_2OP$); 45.35 and 45.15 ($CH_2N$); 42.25/41.91 (CH); 25.59, 22.89; 16.83 ($CH_3C$-O-P).

Example 8 Disodium 3-(guanin-9-ylmethyl)-4-hydroxybutanephosphonate

VSC 660

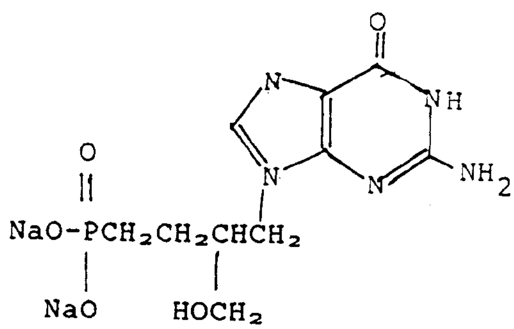

A solution of 2-amino-6-chloro-9-[(2-ethoxy-2-oxo-1,2-oxaphosphorinan -5-yl)methyl]purine (VSC 655; 102 mg, 0,295 mmol) in ethanol (2 ml), water (2 ml), and 2M aqueous sodium hydroxide (1.0 ml, 2 mmol) was kept at 80°C for 3 days, neutralized by addition of weakly acidic Amberlite cation exchange resin, filtered, and evaporated to dryness to give disodium 3-(guanin-9-ylmethyl)-4-hydroxybutanephosphonate.

The starting materials for examples 7 and 8 were prepared as follows:

2-(Acetoxymethyl)-4-bromobutyl acetate

VSC 647

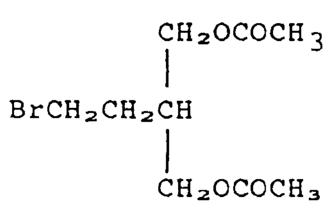

This intermediate was synthesized from 4-(acetoxy)-3-(acetoxymethyl)-butanol according to Literature Procedure. Yield 97% after flash chromatograpy on silica (ethyl acetate + n-hexane 1+1).
TLC $R_f$ 0.67 ($SiO_2$, ethyl acetate + n-hexane 1+1).
$^{13}C$ NMR ($CDCl_3$, TMS, 50 MHz): δ 170.30 (COO); 63.44 ($CH_2O$); 36.27 (CH); 31.67 (Br-$CH_2$); 30.29 (Br-C-$CH_2$); 20.51 ($CH_3$).

Diethyl 4-acetoxy-3-(acetoxymethyl)butanephosphonate

VSC 648

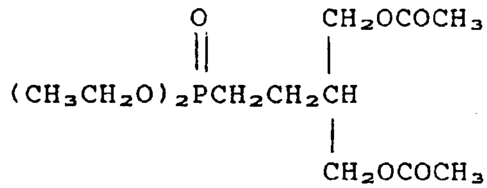

Triethyl phosphite (2.70g, 16.3 mmol) was added with stirring to 2-(acetoxymethyl)-4-bromobutyl acetate (VSC 647, 3.95 g, 14.8 mmol) at 180-190°C and stirring was continued at 190°C for 0.5h. The residue was evaporated in vacuum and kept at ca. 0.1 mB. Flash chromatography on silica with ethyl acetate + ethanol (9+1) yielded 3.36g (70%) of product.
TLC $R_f$ 0.57 ($SiO_2$, ethyl acetate + ethanol 9+1).
$^{13}C$ NMR ($CDCl_3$, TMS, 50 MHz): δ 170.37 (COO); 63.22 ($CH_2OAc$); 61.30/61.18 ($CH_2OP$, J 6 Hz); 37.58/37.27 (CH, J 16 Hz); 24.11/21.29 ($CH_2$-C-P, J 142 Hz); 20.97/20.90 ($CH_2P$ J 4 Hz); 20.46 ($CH_3COO$); 16.20/16.08, J 6 Hz.

(2-Ethoxy-2-oxo-1,2-oxaphosphorinan-5-yl)methanol (racemic cis-trans mixture)

VSC 650

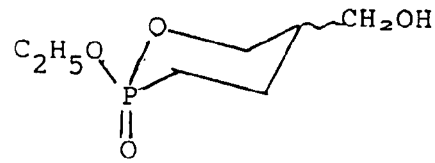

VSC 648 (1.05; 3.24 mmol) was dissolved in 16 ml of a 0.5 molar solution of sodium ethoxide in ethanol. The solution was warmed to 50°C and then kept at 37°C for 2 h. After evaporation to dryness in vacuum, the residue was extracted with ethyl acetate and purified by flash chromatography on silica with ethyl acetate + ethanol (9+1) as eluent. Yield 0.462 g (74%) of VSC 650 in an isomeric (cis-trans) ratio of 0.36/1.00.
TLC $R_f$ 0.26 ($SiO_2$; ethyl acetate + ethanol 9+1).
$^{13}C$ NMR ($CDCl_3$, TMS, 50 MHz); major isomer - minor isomer): δ 72.22/72.08 - 70.64/70.52 ($CH_2O$ in ring, J 6 Hz); 62.18 - 63.64 ($CH_2OH$); 60.81/60.69 - 61.35/61.23 ($CH_2$-O-P, J 6 Hz); 38.87/38.75 - 37.39/37.27 (CH, J 6 Hz); 24.69/24.52 - 23.35/23.18 ($CH_2$-P, J 8 Hz); 23.06/20.48 - 21.38/18.80 ($CH_2$-C-P, J 129 Hz); 16.25/16.15 ($CH_3$-C-O-P, J 5 Hz).

5-(Bromoethyl)-2-ethoxy-2-oxo-1,2-oxaphosphorinane (racemic cis-trans mixture

VCS 654

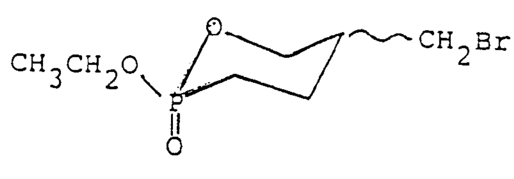

N-Bromosuccinimide (2.67g, 15 mmol) was added in portions to a stirred, ice-chilled solution of 2-ethoxy-2-oxo-1,2-oxaphosphorinan-5-yl)methanol (VSC 650, 1.944 g, 10 mmol) and triphenylphosphine (3.97 g, 15 mmol) in 40 ml of dichloromethane, and stirring was continued for 16h at 4°C. After evaporation in vacuum, diethyl ether (50 ml) was added and the mixture shaken and stirred vigorously. The crystallized triphenylphosphine oxide was removed by filtration and washed with several portions of ether. The combined extracts were evaporated to dryness and purified by flash chromatography on silica with ethyl acetate + ethanol (9+1). Yield 1.569 g (61%) of VSC 654 in a cis-trans ratio of 0.7/1.0. TLC Rf 0.57 ($SiO_2$ ethyl acetate + ethanol 9+1).
$^{13}C$ NMR ($CDCl_3$, TMS, 50 MHz, major isomer - minor isomer): δ 71.76/71.61 ($CH_2O$ in ring, J 6 Hz); 60.74/60.62-61.20/61.08 ($CH_2$-O-P, J 16 Hz); 37.56/37.44-37.10/36.98 (CH, J 6 Hz); 32.13-31.67 ($CH_2Br$); 26.66/26.49-25.37/25.23 ($CH_2P$, J 7 Hz) 22.60/20.02-20.90/18.32 ($CH_2$-C-P, J 129.4 Hz); 16.13/16.01 ($CH_3$-C-O-P, J 6.1 Hz).

2-Amino-6-chloro-9-[(2-ethoxy-2-oxo-1,2-oxaphosphorinan-5-yl)methyl] purine and 7 isomer

VSC 655

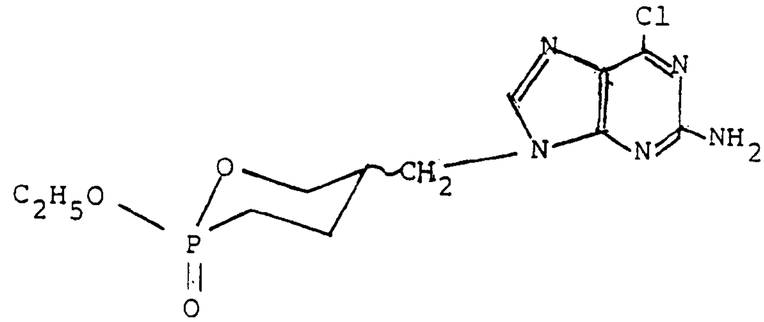

A mixture of 5-(bromoethyl)-2-ethoxy-2-oxo-1,2-oxaphosphorinane (VSC 654; 0.353 g, 1.82 mmol), 2-amino-6-chloropurine (0.50, 2.95 mmol), anhydrous potassium carbonate (0.50 g, 3.62 mmol), and DMF (15 ml) was stirred at room temperature for seven days. Chloroform (30 ml) was added, and after filtration, the solution was evaporated to small volume in vacuum. The residue was purified by flash chromatography on silica (chloroform + methanol 5+1). Yield 0.282 g (45%) as a cis-trans and 7-9 isomeric mixture.

TLC $_{Rf}$ 0.74 and 0.68 for 7 and 9 isomer, respectively ($SiO_2$, chloroform + methanol 5+1).

1H NMR ($CDCl_3$, TMS, 200 MHz): δ 7.80 and 7.76 (s, H8, 7 and 9 isomer); 5.4 (broad s, $NH_2$); 4.3-4.1 (m, $CH_2OP$); 4.02 (d, $CH_2N$); 2.5-2.4 and 2.1-1.7 (m, $CHCH_2CH_2P$); 1.37 (dt, $CH_3$- COP).

$^{13}C$ NMR ($CDCl_3$, TMS, 50 MHz): δ 159.38 ($C_2$); 153.93 (C4); 143.50 (C8); 70.91/70.79-69.94/69.79 ($CH_2O$ in ring, J 7 Hz) 61.79 to 61.45 (2 d, $CH_2$-O-P, J 7 Hz); 44.35 and 43.25 ($CH_2N$); 36.68/36.56-35.05/34.93 (CH, J, 6 Hz); 25.88/25.74-24.18/24.04 ($CH_2P$, J 7 Hz); 22.99/20.41-21.16/18.59 ($CH_2$-C-P, J 129 Hz); 16.59/16.47 ($CH_3$-C-O-P, J 6 Hz).

Biological tests

Test I Effect of compounds of the formula I on HIV in H9 cells

Materials and methods: HIV infection of H9 cells

H9 cells, $10^5$ cells per well on a 24 well plate, suspended in 2 ml RPMI-medium containing 10 % fetal calf serum, 100 μg/ml pencillin, 10 μg/ml streptomycin sulfate and 2 μg/ml polybrene are exposed to HIV (HTLV-IIIB) and different concentrations of the test compounds. The plates are incubated at 37°C in 5 % $CO_2$ for 6-7 days. The contents in each well is then homogenized with a pipette and transferred to a centrifuge tube. After centrifugation for 10 min at 1500 rpm the supernatant is removed and the cell pellet is analyzed by fixing in methanol on glass plates. Human HIV positive serum diluted 1:80 or 1:160 is added and incubated for 30 min at 37°C. The plate is then washed with phosphate-buffered saline (PBS) containing Ca2+ and Mg2+. Sheep antihuman conjugate (FITC) is added and after a new incubation the plate is again washed with PBS. Contrast staining is done with Evans blue and after drying the frequency of HIV antigen containing cells is determined in a microscope. The test result is shown in Table I.

Table I Concentration (μM) for 50 % inhibition ($IC_{50}$) of human immuno deficiency virus multiplication in cell culture

| Compounds | $IC_{50}$ M |
|---|---|
| 9-[4-hydroxy-2-(hydroxymethyl)butyl]-guanine (VSA 671) | 1-10 |
| (-)-9-[4-hydroxy-2-(hydroxymethyl)butyl]-guanine (VSB 647) | 0.1-7 |
| (+)-9-[4-hydroxy-2(hydroxymethyl)butyl]-guanine (VSB 648) | 1-5 |
| 9-[4-hydroxy-2-(hydroxymethyl)butyl]-adenine (VSL 600) | 10 |

Table I shows that the tested compounds are active inhibitors of HIV virus multiplication.

Test II Cellular toxicity

H9 cells, $2x10^7$ cells per plate, are incubated in RPMI-1640 medium containing 10 % fetal calf serum, 70 mg/l penicillin, 100 mg/l streptomycin and 10 mM hepes, in absence or presence of test compounds. The number of cells per plate is determined after 48 h. Cells incubated in the absence of test compounds then underwent two cell division cycles.

F5000 cells, which are human embryo cells, $1x10^5$ cells per plate, are incubated in Eagle's minimal essential medium, supplemented with Earle's salts, non-essential amino acids, 10 % fetal calf serum, 10 mM hepes, 70 mg/l penicillin and 10 mg/l streptomycin, in absence or presence of test compounds. The number of cells per plate is determined after 48 h. Cells incubated in the absence of test compounds underwent one cell division cycle. The results are given as % inhibition of cell multiplication when the concentration of the compounds is 100 μM or 250 μM.

Table II Cellular toxicity on H9 and F5000 cells

| Compound | % inhibition (concentration μM) H9 | F5000 |
|---|---|---|
| 9-[4-hydroxy-2-(hydroxymethyl)-butyl]guanine (VSA 671) | 55 (500) | 55(1000) |
| (-)-9-[4-hydroxy-2-(hydroxymethyl)-butyl]guanine (VSB 647) | 5 (100) | |
| 9-[4-hydroxy-2-(hydroxymethyl) butyl]-adenine (VSC 600) | 25 (200) | 25 (500) |
| 2-(2-aminopurin-9-yl)methyl-butan-1,4-diol (VSB 212) | | 20 (500) |
| | | 75 (500) |

Table II shows that the concentrations at which the compounds exhibit toxicities, vastly exceed the concentrations needed for 50 % inhibition of HIV multiplications as given in Table I.

Test III Oral bioavailability

Oral bioavailability was determined by dosing the animals (cynomologous monkeys and rats) intravenously and orally on separate occasions with the compounds. Blood samples were taken after appropriate intervals for determination of drug level in plasma. Appropriate pharmacokinetic calculations were then carried out based on plasma concentration against time relationship.

Table III Oral bioavailability of compound determined as VSA 671

| Compound | F* % |
|---|---|
| Monkey | |
| 9-[4-hydroxy-2-(hydroxymethyl)butyl]guanine (VSA 671) | 10 |
| 2-(2-aminopurin-9-yl)methylbutane-1,4-diol diacetate (VSC 610) | 32 |
| Rat | |
| 9-[4-hydroxy-2-(hydroxymethyl)butyl]guanine (VSA 671) | 11 |
| 9-[4-acetoxy-2-(acetoxymethyl)butyl]guanine.HCl (VSC 640) | 20 |
| 9-[4-propionoxy-2-(propionoxymethyl)butyl]guanine.HCl (VSC 641) | 19 |
| 2-(2-aminopurin-9-yl)methylbutan-1,4-diol (VSB 212) | 26 |

* Plasma AUC (area under curve) of compound relative to AUC after intravenously given VSA 671.

From the table can be seen how the plasma concentration of VSA 671 is significantly increased after VSA 671 has been given as a 6-deoxy prodrug (VSB 212), and ester (VSC 640, VSC 641) or an ester of 6-deoxy prodrug (VSC 610).

## Claims

**Claims for the following Contracting States : AT, BE, CH, DE, FR, GB, IT, LI, LU, NL, SE**

1. A compound of the formula

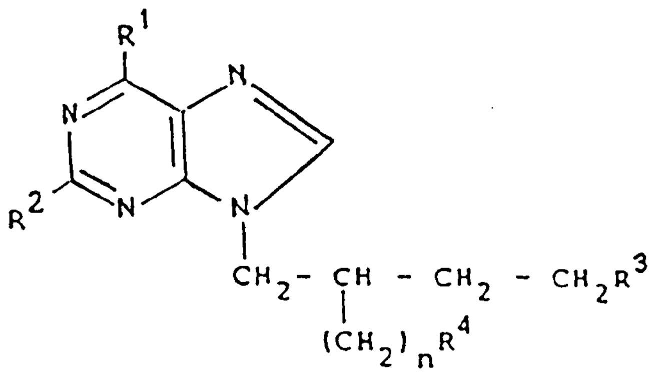

I

wherein:

$R^1$ is hydrogen, hydroxy, mercapto or amino;

$R^2$ is hydrogen, hydroxy, fluoro, chloro or amino;

$R^3$ and $R^4$ are independently selected from

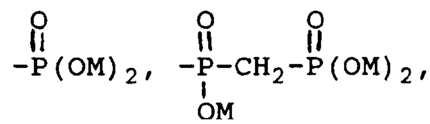

amino, hydroxy

or an ester residue thereof defined as $OR^5$, wherein $R^5$ is $C_{1-6}$ alkyl, arylalkyl, optionally substituted with one or more alkoxy, amino, nitrile or sulphonamido groups or one or more halogen atoms or an ester residue thereof derived from a carboxylic acid $R^6COOH$, a carbonic acid $R^7OCOOH$, a double ester of a carbonic acid $R^7CO$-$OCH(R^8)OCOOH$, a sulphonic acid $R^7SO_2OH$, a carbamic acid $R^7NHCOOH$, or a phosphoric acid, wherein $R^6$ is hydrogen, $C_{1-17}$ alkyl, alkoxyalkyl, arylalkyl or aryl, $R^7$ is $C_{1-17}$ alkyl, arylalkyl or aryl, $R^8$ is hydrogen or $C_{1-13}$ alkyl and said aryl and arylalkyl groups optionally can be substituted with one or more alkyl, alkoxy, amino, nitrile, sulphonamido groups or one or more halogen atoms ,or $R^3$ together with $R^4$ is

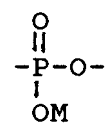

wherein

M is hydrogen or a pharmaceutically acceptable counterion; and n is 1 or 2; with the proviso that, when $R^2$ is amino and $R^3$ and $R^4$ are hydroxy, $R^1$ is not hydroxy and in addition, when n = 1, $R^1$ is not hydrogen, and pharmaceutically acceptable salts thereof.

2. A compound according to claim 1 in the form of an optical isomer.

3. A compound according to claim 1 or 2, wherein $R^3$ and $R^4$ are both hydroxy.

4. A compound according to claim 1 or 2, wherein $R^3$ is

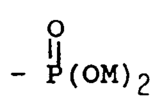

and $R^4$ is OH or

$R^3$ together with $R^4$ is

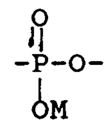

5. A compound according to any of claims 1-4 for use in therapy.

6. A compound of the formula I as defined in any of claims 1-4 for therapeutic and/or prophylactic treatment of virus infections in an animal or human host in the need of treatment.

7. A compound according to claim 6 for treatment of infections caused by herpes viruses.

8. A compound according to claim 6 for treatment of infections caused by viruses requiring reverse transcriptase for replication, including human immuno deficiency viruses and hepatitis B virus.

9. A pharmaceutical composition comprising as active ingredient a compound according to any of claims 1-4 and a pharmaceutically acceptable carrier.

10. A process for preparation of a compound of the formula

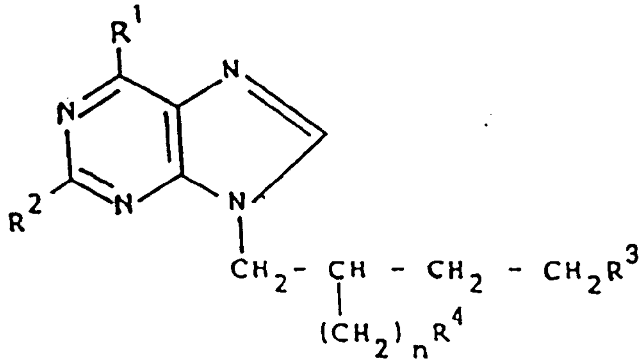

I

wherein $R^1$, $R^2$, $R^3$, $R^4$ and n are as defined in claim 1, by

A. condensing an acyclic side chain

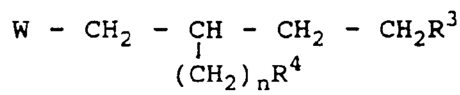

where W is a terminal leaving group, to the N-9 position of a purine derivative

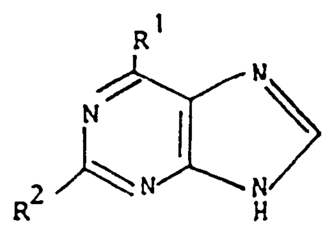

B. imidazole ring closure of a pyrimidine derivative thereof of the formula

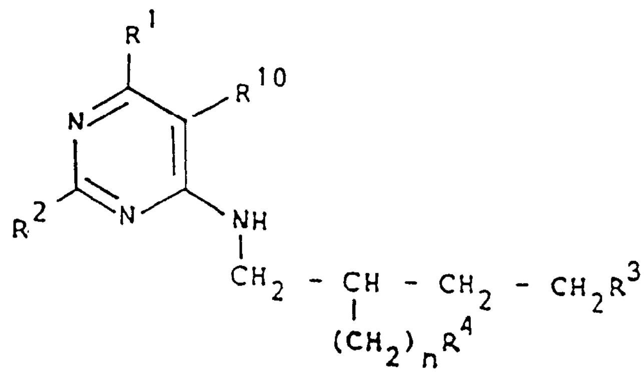

wherein R is amino or an amino derivative;
C. imidazole ring closure of a furazano-[3,4-d]pyrimidine ring of the formula

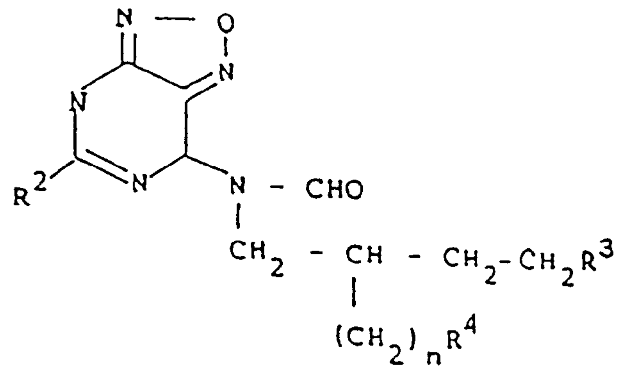

and reductive cleavage of the furazane ring to a compound of the formula I, wherein $R^1$ is amino; or
D. pyrimidine ring closure of an imidazole derivative of the formula

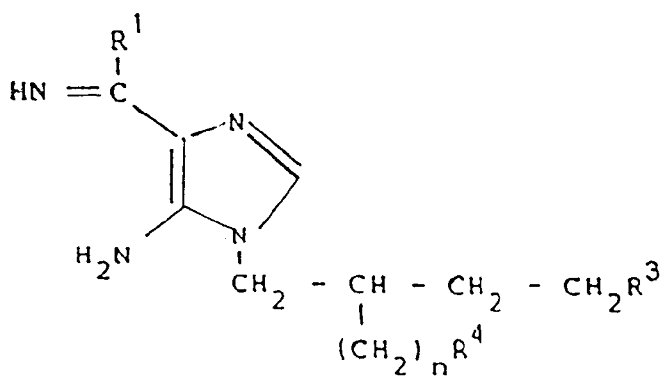

in which processes $R^1$ - $R^4$ and n are as defined in claim 1 and optionally may be protected by suitable protecting groups, whereby a mixture of optical isomers or a single optical isomer is obtained and a racemic mixture obtained is optionally separated into the optical isomers.
11. Use of a compound of the formula I

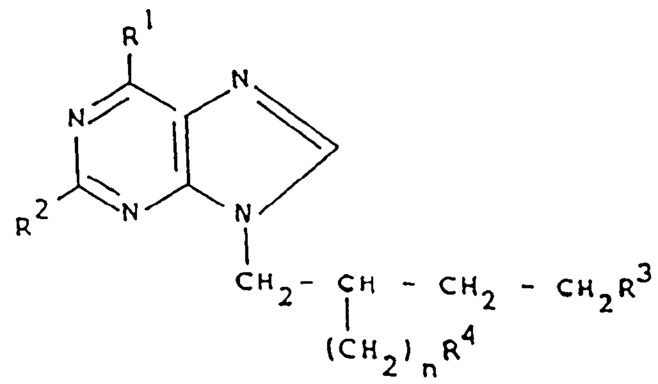

I

wherein:
$R^1$ is hydrogen, hydroxy, mercapto or amino;
$R^2$ is hydrogen, hydroxy, fluoro, chloro or amino;
$R^3$ and $R^4$ are independently selected from

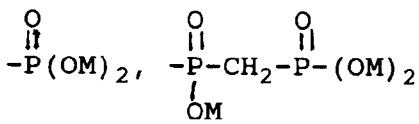

amino, hydroxy
or an ether residue thereof defined as $OR_5$, wherein $R^5$ is $C_{1-6}$ alkyl, arylalkyl optionally substituted with one or more alkoxy, amino, nitrile, or sulphonamido groups or one or more halogen atoms,
or an ester residue thereof derived from a carboxylic acid $R^6COOH$, a carbonic acid $R^7OCOOH$, a double ester of a carbonic acid $R^7COOCH(R^8)OCOOH$, a sulphonic acid $R^7SO_2OH$, a carbamic acid $R^7NHCOOH$, or a phosphoric acid, wherein $R^6$ is hydrogen, $C_{1-17}$ alkyl, alkoxyalkyl, arylalkyl or aryl, $R^7$ is $C_{1-17}$ alkyl, arylalkyl or aryl, $R^8$ is hydrogen or $C_{1-3}$ alkyl and said aryl and arylalkyl groups optionally can be substituted with one or more alkyl, alkoxy, amino, nitrile, sulphonamido groups or one or more halogen atoms or $R^3$ together with R4 is

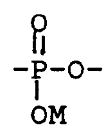

wherein
M is hydrogen or a pharmaceutically acceptable counterion; and n is 1 or 2; and pharmaceutically acceptable salts thereof for the manufacture of a medicament for therapeutic and/or prophylactic treatment of the acquired immuno deficiency syndrome and infections caused by viruses requiring reverse transcriptase for replication.

12. Use of a compound of the formula according to claim 11 in the form of an optical isomer.

13. Use according to claim 11 or 12 for the treatment of infections caused by HIV-viruses.

14. Use according to claim 11 or 12 for the treatment of infections caused by hepatitis B viruses.

**Claims for the following Contracting States : ES, GR**

1. A process for the preparation of a compound of the formula

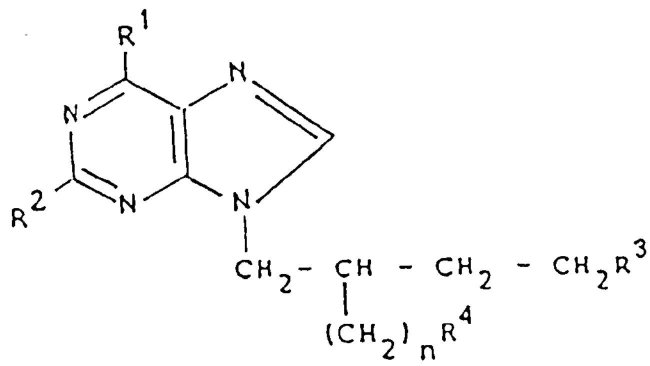

I

wherein:

$R^1$ is hydrogen, hydroxy, mercapto or amino;
$R^2$ is hydrogen, hydroxy, fluoro, chloro, or amino;
$R^3$ and $R^4$ are independently selected from

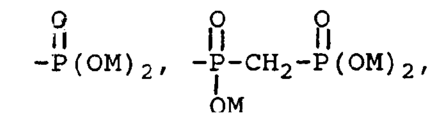

amino, hydroxy
or an ether residue thereof defined as $OR^5$, wherein $R^5$ is $C_{1-6}$ alkyl, arylalkyl optionally substituted with one or more alkoxy amino, nitrile or sulphonamido group or one or more halogen atoms or an ester residue thereof derived from a carboxylic acid $R^6COOH$, a carbonic acid $R^7OCOOH$, a double ester of a carbonic acid $R^7CO$-$OCH(R^8)OCOOH$, a sulphonic acid $R^7SO_2OH$, a carbamic acid $R^7NHCOOH$, or a phosphoric acid, wherein $R^6$ is hydrogen, $C_{1-17}$ alkyl, alkoxyalkyl, arylalkyl or aryl, $R^7$ is $C_{1-17}$ alkyl, arylalkyl or aryl, $R^8$ is hydrogen or $C_{1-13}$ alkyl and said aryl and arylalkyl groups optionally can be substituted with one or more alkyl, alkoxy, amino, nitrile, sulphonamido groups or one or more halogen atoms, or $R^3$ together with $R^4$ is

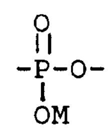

wherein
M is hydrogen or a pharmaceutically acceptable counterion; and n is 1 or 2; with the proviso that, when $R^2$ is amino and $R^3$ and $R^4$ are hydroxy, $R^1$ is not hydroxy and in addition, when n = 1, $R^1$ is not hydrogen, and pharmaceutically acceptable salts thereof, by

A. condensing an acyclic side chain

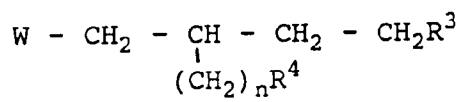

where W is a terminal leaving group, to the N-9 position of a purine derivative

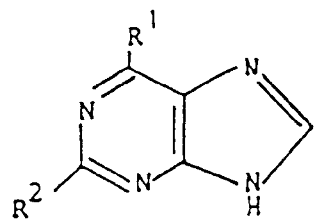

B. imidazole ring closure of a pyrimidine derivative thereof of the formula

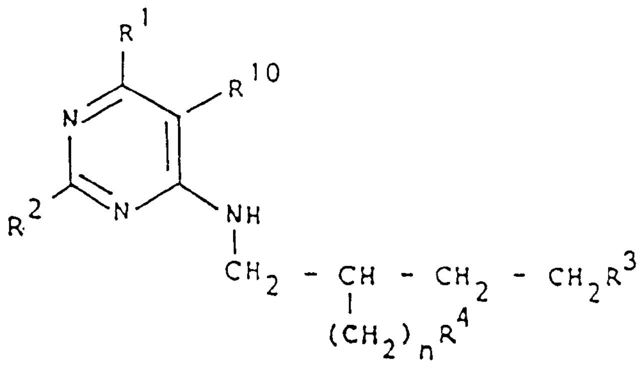

wherein R is amino or an amino derivative;
C. imidazole ring closure of a furazano-[3,4-d]pyrimidine ring of the formula

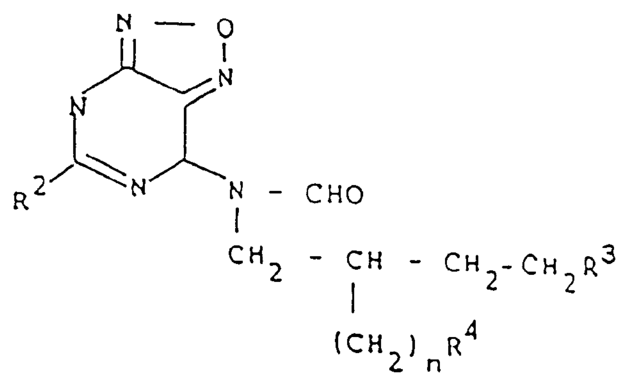

and reductive cleavage of the furazane ring to a compound of the formula I, wherein $R^1$ is amino; or
D. pyrimidine ring closure of an imidazole derivative of the formula

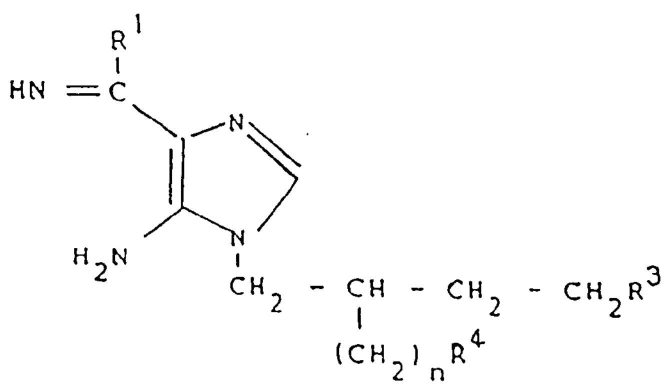

in which processes $R^1$ - $R^4$ and n are as defined above and optionally may be protected by suitable protecting groups, whereby a mixture of optical isomers or a single optical isomer is obtained and a racemic mixture obtained is optionally separated into the optical isomers.

2. A process according to claim 1 for preparation of a compound of the formula I in the form of an optical isomer.

3. A process according to claim 1 or 2 for preparation of a compound of the formula I, wherein $R^3$ and $R^4$ are both hydroxy.

4. A process according to claim 1 or 2 for the preparation of a compound of the formula I wherein

$R^3$ is

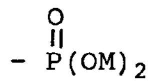

and $R^4$ is OH or

$R^3$ together with $R^4$ is

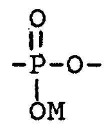

5. A process for preparing a compound according to any of claims 1-4 for use in therapy.

6. A process for preparing a compound of the formula I as defined in any of claims 1-4 for therapeutic and/or prophylactic treatment of virus infections in an animal or human host in the need of treatment.

7. A process for preparing a compound according to claim 6 for treatment of infections caused by herpes viruses.

8. A process for preparing a compound according to claim 6 for treatment of infections caused by viruses requiring reverse transcriptase for replication, including human immuno deficiency viruses and hepatitis B virus.

9. A process for preparing a pharmaceutical composition comprising combining, as active ingredient, a compound according to any of claims 1-4 with a pharmaceutically acceptable carrier.

10. Use of a compound of the formula I

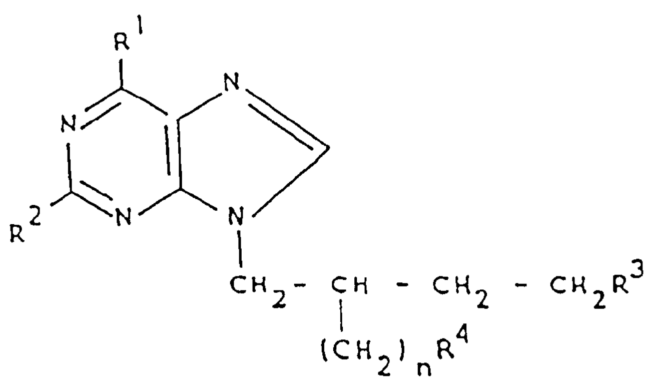

I

wherein:

$R^1$ is hydrogen, hydroxy, mercapto or amino;

$R^2$ is hydrogen, hydroxy, fluoro, chloro or amino;

$R^3$ and $R^4$ are independently selected from

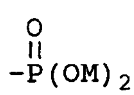 , 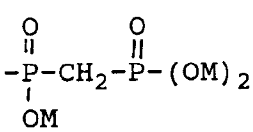

amino, hydroxy
or an ether residue thereof defined as $OR_5$, wherein $R^5$ is $C_{1-6}$ alkyl, arylalkyl optionally substituted with one or more alkoxy, amino, nitrile or sulphonamido groups or one or more halogen atoms,
or an ester residue thereof derived from a carboxylic acid $R^6COOH$, a carbonic acid $R^7OCOOH$, a double ester of a carbonic acid $R^7COOCH(R^8)OCOOH$, a sulphonic acid $R^7SO_2OH$, a carbamic acid $R^7NHCOOH$, or a phosphoric acid, wherein $R^6$ is hydrogen, $C_{1-17}$ alkyl, alkoxyalkyl, arylalkyl or aryl, $R^7$ is $C_{1-17}$ alkyl, arylalkyl or aryl, $R^8$ is hydrogen or $C_{1-3}$ alkyl and said aryl and arylalkyl groups optionally can be substituted with one or more alkyl, alkoxy, amino, nitrile, sulphonamido groups or one or more halogen atoms or $R^3$ together with R4 is

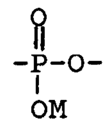

wherein
M is hydrogen or a pharmaceutically acceptable counterion; and n is 1 or 2; and pharmaceutically acceptable salts thereof for the manufacture of a medicament for therapeutic and/or prophylactic treatment of the acquired immuno deficiency syndrome and infections caused by viruses requiring reverse transcriptase for replication.

11. Use of a compound of the formula according to claim 10 in the form of an optical isomer.
12. Use according to claim 10 or 11 for the treatment of infections caused by HIV-viruses.
13. Use according to claim 10 or 11 for the treatment of infections caused by hepatitis B viruses.

## Patentansprüche

### Patentansprüche für folgende Vertragsstaaten : AT, BE, CH, DE, FR, GB, IT, LI, LU, NL, SE

1. Verbindung der Formel I

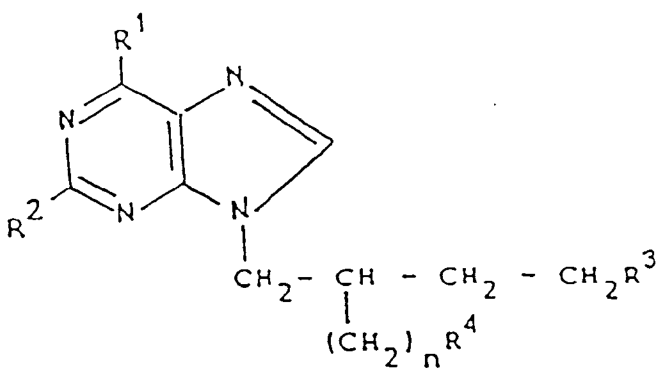

worin sind:
$R^1$ ein Wasserstoffatom, eine Hydroxy-, Mercapto- oder Aminogruppe;
$R^2$ ein Wasserstoffatom, eine Hydroxygruppe, ein Fluoratom, ein Chloratom oder eine Aminogruppe;
$R^3$ und $R^4$ voneinander unabhängig ausgewählt aus

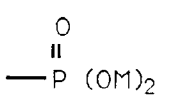
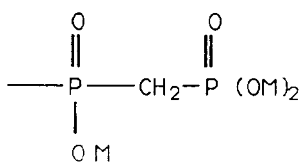

Amino-, Hydroxy- oder einem Etherrest,
definiert als $OR^5$, worin $R^5$ ein $C_{1-6}$-Alkyl, ein Arylalkyl - wahlweise substituiert mit ein oder mehreren Alkoxy-, Amino-, Nitril- oder Sulphonamidgruppen oder mit ein oder mehreren Halogenatomen oder einem Esterrest

davon, der ableitbar ist aus einer Carbonsäure $R^6COOH$, einer Kohlensäureesterverbindung $R^7OCOOH$, einem Doppelester der Kohlensäure $R^7COOCH(R^8)OCOOH$, einer Sulphonsäure $R^7SO_2OH$, einer Carbaminsäure $R^7NHCOOH$, einer Phosphorsäure, worin,sind: $R^6$ gleich Wasserstoff, $C_{1-17}$-Alkyl, Alkoxyalkyl, Arylalkyl oder Aryl; $R^7$ ein $C_{1-17}$-Alkyl, Arylalkyl oder Aryl, $R^8$ ein Wasserstoffatom oder ein $C_{1-13}$-Alkyl, wobei die Aryl- und Arylalkylgruppen wahlweise mit ein oder mehreren Alkyl-, Alkoxy-, Amino-, Nitril-, Sulphonamidgruppen oder ein oder mehreren Halogenatomen substituiert sein können, oder $R^3$ zusammen mit $R^4$ ein

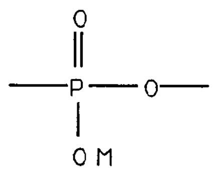

sind, worin
M ein Wasserstoffatom oder ein pharmazeutisch verwendbares Gegenion ist;
und n = 1 oder 2; unter den Voraussetzungen: wenn $R^2$ eine Aminogruppe und $R^3$ und $R^4$ Hydroxygruppen sind, daß dann $R^1$ keine Hydroxygruppe ist und zusätzlich, wenn n gleich 1 ist, daß $R^1$ kein Wasserstoffatom ist; sowie pharmazeutisch akzeptable Salze davon.

2. Verbindung gemäß Anspruch 1 in der Form eines optischen Isomers.

3. Verbindung gemäß Anspruch 1 oder 2, worin $R^3$ und $R^4$ beide Hydroxygruppen sind.

4. Verbindung gemäß Anspruch 1 oder 2, worin sind:
$R^3$ gleich

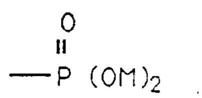

und $R^4$ ein OH oder
$R^3$ zusammen mit $R^4$ ein

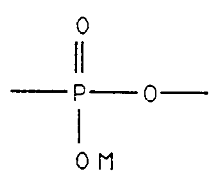

5. Verwendung einer Verbindung gemäß einem der Ansprüche 1 bis 4 in der Therapie.

6. Verbindung der Formel I gemäß einem der Ansprüche 1 bis 4 zur therapeutischen und/oder prophylaktischen Behendlung von Virusinfektionen bei behandlungsbedürftigen Tieren oder Menschen.

7. Verbindung gemäß Anspruch 6 zur Behandlung von Infektionen aufgrund von Herpes-Viren.

8. Verbindung gemäß Anspruch 6 zur Behandlung von Infektionen weger Viren, die zur Replikation reverse Transkriptase benötigen, einschließlich des menschlichen Immunschwäche-viruses und des Hepatits-B-Viruses.

9. Pharmazeutische Zusammensetzung, die als Wirksubstanz eine Verbindung gemäß einem der Ansprüche 1 bis 4 enthält sowie einen pharmazeutisch akzeptablen Träger.

10. Verfahren zur Herstellung einer Verbindung der Formel I

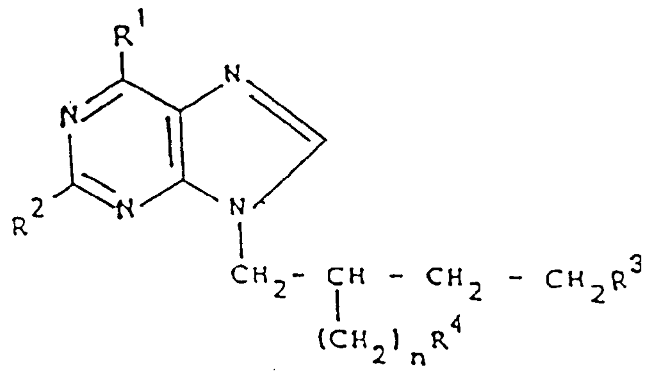

worin sind $R^1$, $R^2$, $R^3$, $R^4$ und n wie in Anspruch 1 definiert, durch

A. Kondensation einer acylischen Seitenkette

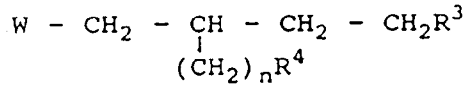

worin W eine terminale Abgangsgruppe ist, an die N-9-Position des Purinderivates

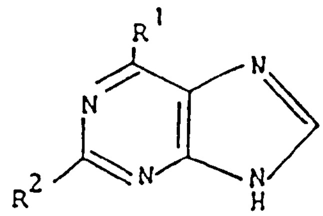

B. Imidazol-Ringschluß des Pyrimidinderivats der Formel

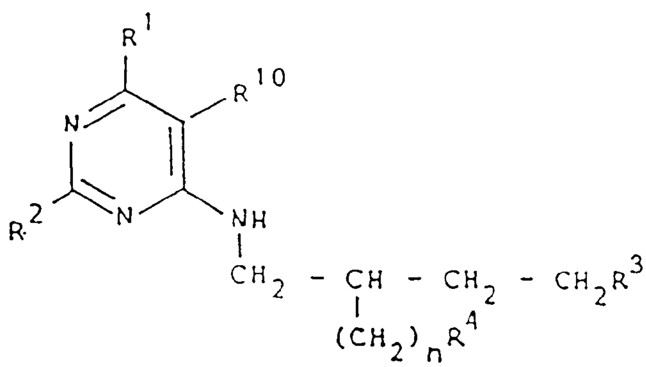

worin R eine Aminogruppe ist oder ein Aminoderivat;

C. Imidazol-Ringschluß des Furazano-[3,4-d]pyrimidin-Rings der Formel

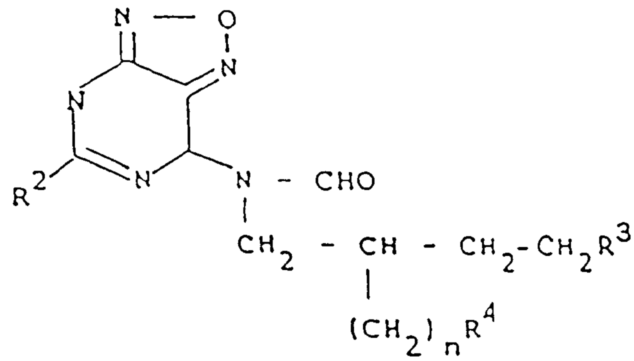

und Reduktionsspaltung des Furazanringes der Verbindung der Formel I, worin $R^1$ eine Aminogruppe ist; oder

D. Pyrimidin-Ringschluß eines Imidazol-Derivates der Formel

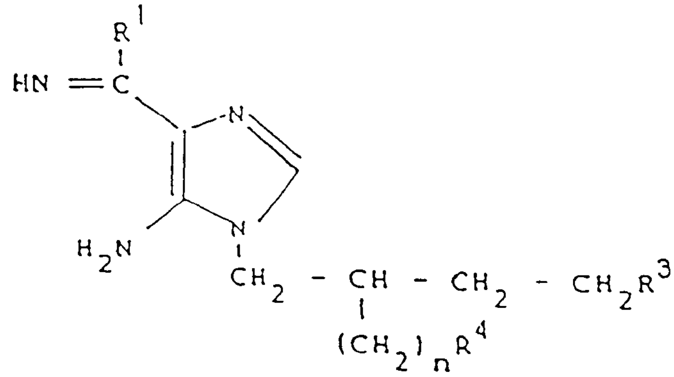

worin die Fortsätze $R^1$ bis $R^4$ und n wie in Anspruch 1 definiert und wahlweise durch geeignete Schutzgruppen geschützt sind, wobei eine Mischung der optischen Isomere oder ein einziges optisches Isomer erhalten wird und racemische Mischungen wahlweise in die optischen Isomere aufgetrennt werden.

11. Verwendung einer Verbindung der Formel I

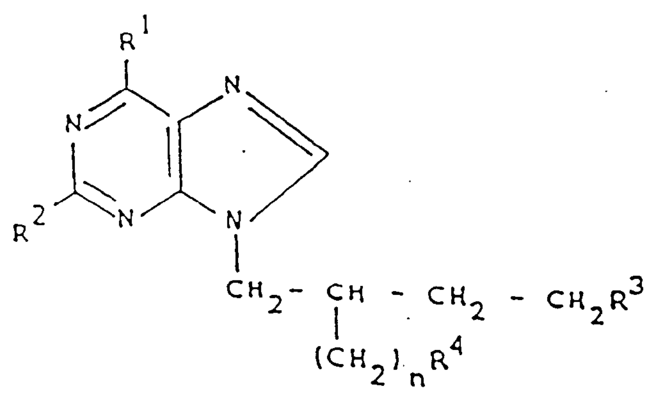

worin sind:

$R^1$ ein Wasserstoffatom, eine Hydroxy-, Mercapto- oder Aminogruppe;

$R^2$ ein Wasserstoffatom, eine Hydroxygruppe, ein Fluor-, Chloratom oder eine Aminogruppe;

$R^3$ und $R^4$ unabhängig voneinander ausgewählt aus

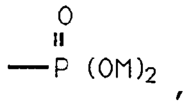 , 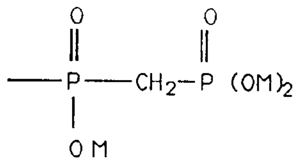

Amino-, Hydroxy- oder einem Etherrest, der definiert ist als $OR^5$, worin $R^5$ ein $C_{1-6}$-Alkyl, ein Arylalkyl, das wahlweise mit ein oder mehreren Alkoxy-, Amino-, Nitril- oder Sulphonamidgruppen oder ein oder mehreren Halogenatomen substituiert sind oder einem Esterrest, der abgeleitet ist aus der Carbonsäure $R^6COOH$, dem Kohlensäureester $R^7OCOOH$, dem zweifachen Kohlensäureester $R^7COOCH(R^8)OCOOH$, der Sulphonsäure $R^7SO_2OH$, der Carbaminsäure $R^7NHCOOH$ oder eine Phosphorsäure, worin sind: $R^6$ ein Wasserstoffatom, $C_{1-17}$-Alkyl, ein Alkoxyalkyl, ein Arylalkyl oder Aryl ist, wobei $R^1$ ein $C_{1-17}$-Alkyl, Arylalkyl oder Aryl ist, $R^8$ ein Wasserstoffatom oder ein $C_{1-3}$-Alkyl ist und die besagten Aryl- und Arylalkylgruppen substituiert sein können mit ein oder mehreren Alkyl-, Alkoxy-, Amino-, Nitril-, Sulphonamid-gruppen oder ein oder mehreren Halogenatomen oder $R^3$ zusammen mit $R^4$ ein

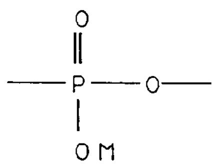 ,

worin M ein Wasserstoffatom oder ein pharmazeutisch akzeptables Gegenion ist; und n gleich 1 oder 2 ist; und pharmazeutisch akzeptable Salze davon, zur Herstellung eines Medikaments zur therapeutischen und/oder prophylaktischen Behandlung des erworbenen Immunschwäche-Syndroms und von Infektionen aufgrund von Viren, die zur Replikation reverse Transkriptase benötigen.

12. Verwendung einer Verbindung der Formel gemäß Anspruch 11 in Form eines optischen Isomers.

13. Verwendung gemäß Anspruch 11 oder 12 zur Behandlung von Infektionen aufgrund von HIV-Viren.

14. Verwendung gemäß Anspruch 11 oder 12 zur Behandlung von Infektionen aufgrund von Hepatis-B-Viren.

**Patentansprüche für folgende Vertragstaaten : ES, GR**

1. Verfahren zur Herstellung einer Verbindung der Formel I

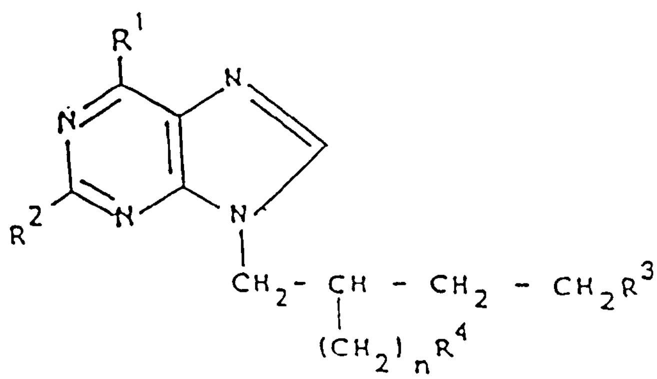

I

worin sind:

$R^1$ ein Wasserstoffatom, eine Hydroxy-, Mercapto- oder Aminogruppe;

$R^2$ ein Wasserstoffatom, eine Hydroxygruppe, ein Fluoratom, ein Chloratom oder eine Aminogruppe;

$R^3$ und $R^4$ voneinander unabhängig ausgewählt aus

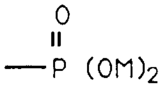
,
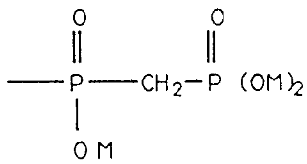

Amino-, Hydroxy- oder einem Etherrest,
definiert als $OR^5$, worin $R^5$ ein $C_{1-6}$-Alkyl, ein Arylalkyl - wahlweise substituiert mit ein oder mehreren Alkoxy-, Amino-, Nitril- oder Sulphonamidgruppen oder mit ein oder mehreren Halogenatomen
oder einem Esterrest davon, der ableitbar ist aus einer Carbonsäure $R^6COOH$, einer Kohlensäureesterverbindung $R^7OCOOH$, einem Doppelester der Kohlensäure $R^7COOCH(R^8)OCOOH$, einer Sulphonsäure $R^7SO_2OH$, einer Carbaminsäure $R^7NHCOOH$, einer Phosphorsäure, worin,sind: $R^6$ gleich Wasserstoff, $C_{1-17}$-Alkyl, Alkoxyalkyl, Arylalkyl oder Aryl; $R^7$ ein $C_{1-17}$-Alkyl, Arylalkyl oder Aryl, $R^8$ ein Wasserstoffatom oder ein $C_{1-13}$-Alkyl, wobei die Aryl- und Arylalkylgruppen wahlweise mit ein oder mehreren Alkyl-, Alkoxy-, Amino-, Nitril-, Sulphonamidgruppen oder ein oder mehreren Halogenatomen substituiert sein können, oder $R^3$ zusammen mit $R^4$ ein

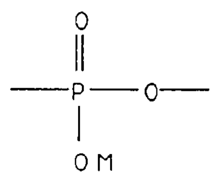

sind, worin
M ein Wasserstoffatom oder ein pharmazeutisch verwendbares Gegenion ist; und n = 1 oder 2; unter den Voraussetzungen: wenn $R^2$ eine Aminogruppe und $R^3$ und $R^4$ Hydroxygruppen sind, daß dann $R^1$ keine Hydroxygruppe ist und zusätzlich, wenn n gleich 1 ist, daß $R^1$ kein Wasserstoffatom ist; sowie pharmazeutisch akzeptable Salze davon. durch

A. Kondensation einer acylischen Seitenkette

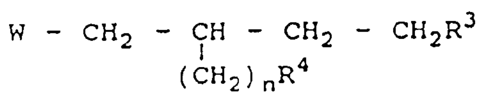

worin W eine terminale Abgangsgruppe ist, an die N-9-Position des Purinderivates

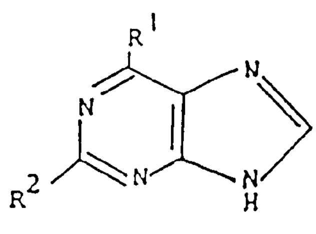

B. Imidazol-Ringschluß des Pyrimidinderivats der Formel

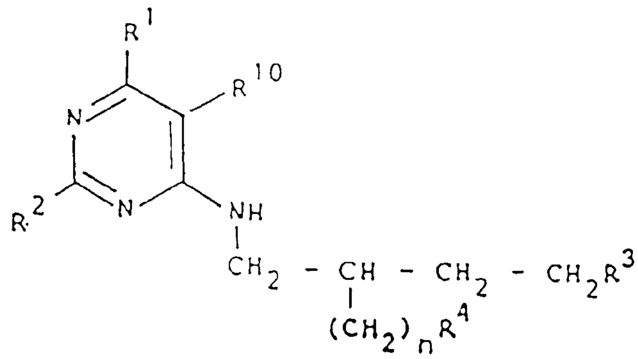

worin R eine Aminogruppe ist oder ein Aminoderivat;

C. Imidazol-Ringschluß des Furazano-[3,4-d]pyrimidin-Rings der Formel

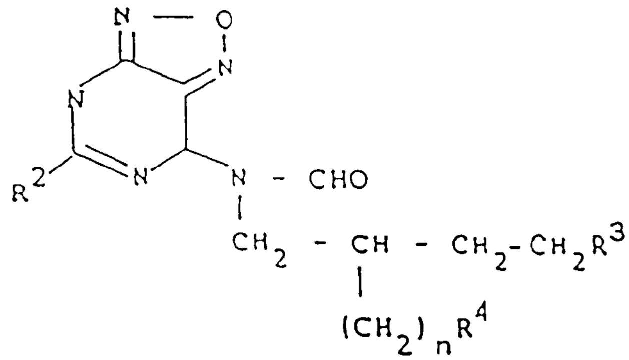

und Reduktionsspaltung des Furazanringes der Verbindung der Formel I, worin $R^1$ eine Aminogruppe ist; oder

D. Pyrimidin-Ringschluß eines Imidazol-Derivates der Formel

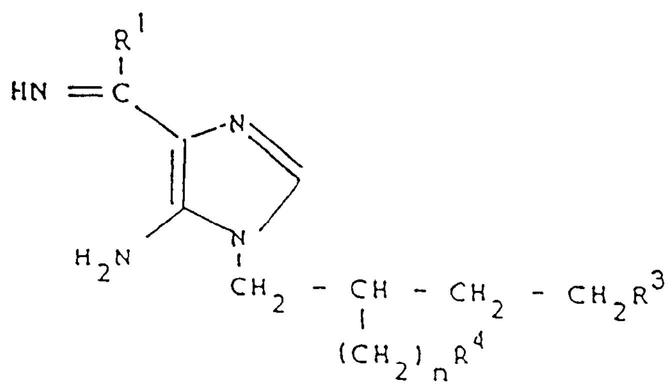

worin die Fortsätze $R^1$ bis $R^4$ und n wie in Anspruch 1 definiert und wahlweise durch geeignete Schutzgruppen geschützt sind, wobei eine Mischung der optischen Isomere oder ein einziges optisches Isomer erhalten wird und racemische Mischungen wahlweise in die optischen Isomere aufgetrennt werden.

2. Verfahren gemäss Anspruch 1 zur Herstellung einer Verbindung der Formel I in der Form eines optischen Isomes.

3. Verfahren gemäss Anspruch 1 oder 2 zur Herstellung einer Verbindung der Formel I, worin $R^3$ und $R^4$ beide Hydroxygruppen sind.

4. Verbindung gemäss Anspruch 1 oder 2, zur Herstellung einer Verbindung der Formel I, worin sind:
$R^3$ gleich

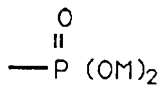

und $R^4$ ein OH oder
$R^3$ zusammen mit $R^4$ ein

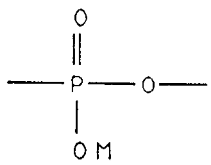

5. Verfahren zur Herstellung einer Verbindung gemäss einem der Ansprüche 1 bis 4 in der Therapie.

6. Verfahren zur Herstellung einer Verbindung der Formel I gemäss einem der Ansprüche 1 bis 4 zur therapeutischen und/oder prophylaktischen Behandlung von Virusinfektionen bei behandlungsbedürftigen Tieren oder Menschen.

7. Verfahren zur Herstellung einer Verbindung gemäss Anspruch 6 zur Behandlung von Infektionen aufgrund von Herpes-Viren.

8. Vervahren zur Herstellung einer Verbindung gemäss Anspruch 6 zur Behandlung von Infektionen wegen Viren, die zur Replikation reverse Transkriptase benötigen, einschliesslich des menschlichen Immunschwächeviruses und des Hepatits-B-Viruses.

9. Verfahren zur Herstellung einer pharmazeutische Zusammensetzung durch Kombination einer Verbindung gemäss einem der Ansprüche 1 bis 4 als Wirksubstanz und eines pharmazeutisch akzeptablen Träger.

10. Verwendung einer Verbindung der Formel I

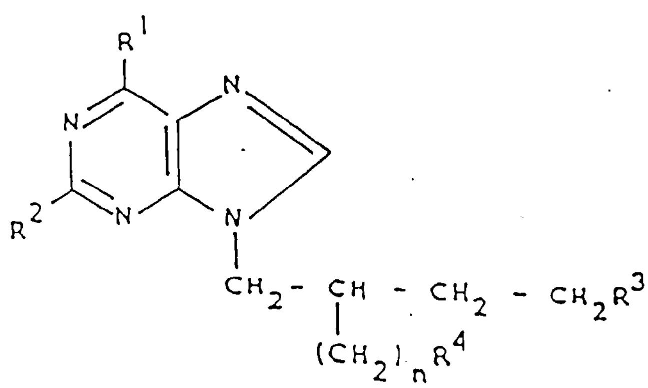

worin sind
$R^1$ ein Wasserstoffatom, eine Hydroxy-, Mercapto- oder Aminogruppe;
$R^2$ ein Wasserstoffatom, eine Hydroxygruppe, ein Fluor-, Chloratom oder eine Aminogruppe;
$R^3$ und $R^4$ unabhängig voneinander ausgewählt aus

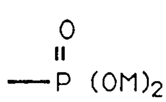 , 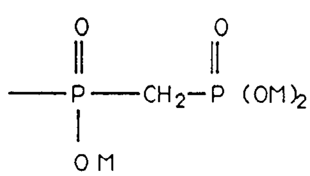

Amino-, Hydroxy- oder einem Etherrest, der definiert ist als $OR^5$, worin $R^5$ ein $C_{1-6}$-Alkyl, ein Arylalkyl, das wahlweise mit ein oder mehreren Alkoxy-, Amino-, Nitril- oder Sulphonamidgruppen oder ein oder mehreren Halogenatomen substituiert sind oder einem Esterrest, der abgeleitet ist aus der Carbonsäure $R^6COOH$, dem Kohlensäureester $R^7OCOOH$, dem zweifachen Kohlensäureester $R^7COOCH(R^8)OCOOH$, der Sulphonsäure

$R^7SO_2OH$, der Carbaminsäure $R^7NHCOOH$ oder eine Phosphorsäure, worin sind: $R^6$ ein Wasserstoffatom, $C_{1-17}$-Alkyl, ein Alkoxyalkyl, ein Arylalkyl oder Aryl ist, wobei $R^7$ ein $C_{1-17}$-Alkyl, Arylalkyl oder Aryl ist, $R^8$ ein Wasserstoffatom oder ein $C_{1-3}$-Alkyl ist und die besagten Aryl- und Arylalkylgruppen substituiert sein können mit ein oder mehreren Alkyl-, Alkoxy-, Amino-, Nitril-, Sulphonamidgruppen oder ein oder mehreren Halogenatomen oder $R^3$ zusammen mit $R^4$ ein

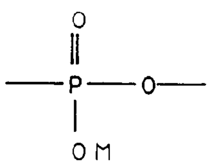

worin M ein Wasserstoffatom oder ein pharmazeutisch akzeptables Gegenion ist; und n gleich 1 oder 2 ist; und pharmazeutisch akzeptable Salze davon, zur Herstellung eines Medikaments zur therapeutischen und/oder prophylaktischen Behandlung des erworbenen Immunschwäche-Syndroms und von Infektionen aufgrund von Viren, die zur Replikation reverse Transkriptase benötigen.

11. Verwendung einer Verbindung der Formel gemäß Anspruch 10, in Form eines optischen Isomers.

12. Verwendung gemäß Aspruch 10 oder 11 zur Behandlung von Infektionen aufgrund von HIV-Viren.

13. Verwendung gemäß Anspruch 10 oder 11 zur Behandlung von Infektionen aufgrund von Hepatis-B-Viren.

## Revendications

### Revendications pour les Etats contractants suivants : AT, BE, CH, DE, FR, GB, IT, LI, LU, NL, SE

1. Composé de formule I:

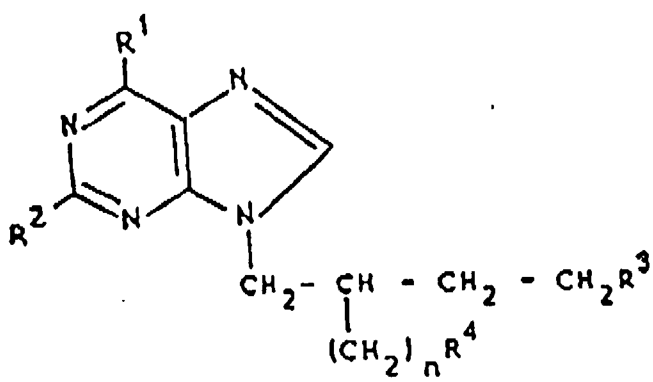

I

dans laquelle:
$R^1$ est un atome d'hydrogène, un groupe hydroxy, mercapto ou amino;
$R^2$ est un atome d'hydrogène, de fluor, de chlore, un groupe hydroxy ou amino;
$R^3$ et $R^4$ sont indépendamment choisis parmi

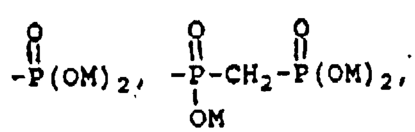

amino, hydroxy,
ou un résidu éther de ceux-ci défini en tant que $OR^5$, dans lequel $R^5$ est un groupe alkyle en $C_{1-6}$, arylalkyle éventuellement substitué avec un ou plusieurs groupes alcoxy, amino, nitrile ou sulfonamido ou un ou plusieurs atomes d'halogènes, ou un résidu ester de ceux-ci dérivé d'un acide carboxylique $R^6COOH$, d'un acide car-

bonique $R^7OCOOH$, d'un ester double d'un acide carbonique $R^7CO_2CH(R^8)OCO_2H$, d'un acide sulfonique $R^7SO_2OH$, d'un acide carbamique $R^7NHCOOH$ ou d'un acide phosphorique, dans lesquels $R^6$ est un atome d'hydrogène, un groupe alkyle en $C_{1-17}$, alcoxyalkyle ou aryle, $R^7$ est un groupe alkyle en $C_{1-17}$, arylalkyle ou aryle, $R^8$ est un atome d'hydrogène ou un groupe alkyle en $C_{1-13}$ et ces groupes aryles et arylalkyles peuvent éventuellement être substitués avec un ou plusieurs groupes alkyle, alcoxy, nitrile ou sulfonamide ou un ou plusieurs atomes d'halogène,
ou $R^3$ forme avec $R^4$ un groupe

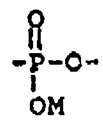

dans lequel
M est un atome d'hydrogène ou un contre-ion acceptable du point de vu pharmaceutique;
et n est 1 ou 2; à condition que, lorsque $R^2$ est un groupe amino et que $R^3$ et $R^4$ sont un groupe hydroxy, $R^1$ ne soit pas un groupe hydroxy et que de plus, lorsque n = 1, $R^1$ ne soit pas un atome d'hydrogène, et des sels de celui-ci acceptables du point de vue pharmaceutique.

2. Composé suivant la revendication 1 sous la forme d'un isomère optique.

3. Composé suivant la revendication 1 ou la revendication 2, dans lequel $R^3$ et $R^4$ sont tous les deux un groupe hydroxy.

4. Composé suivant la revendication 1 ou la revendication 2, dans lequel $R^3$ est

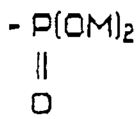

et $R^4$ est OH,
ou $R^3$ forme avec $R^4$ un groupe

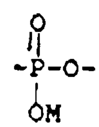

5. Composé suivant l'une quelconque des revendication 1 à 4, pour l'utilisation en thérapie.

6. Composé de formule I suivant l'une quelconque des revendications 1 à 4, pour l'utilisation dans le traitement thérapeutique et/ou prophylactique des infections virales chez un hôte animal ou humain nécessitant un traitement.

7. Composé suivant la revendication 6 pour le traitement d'infections provoquées par les virus de l'herpès.

8. Composé suivant la revendication 6 pour le traitement d'infections provoquées par des virus nécessitant une reverse transcriptase pour leur réplication, comprenant les virus d'immunodéficience humaine et le virus de l'hépatite B.

9. Composition pharmaceutique comprenant en tant que composant actif, un composé suivant l'une quelconque des revendications 1 à 4 et un support acceptable du point de vue pharmaceutique.

10. Procédé pour la préparation d'un composé de formule I

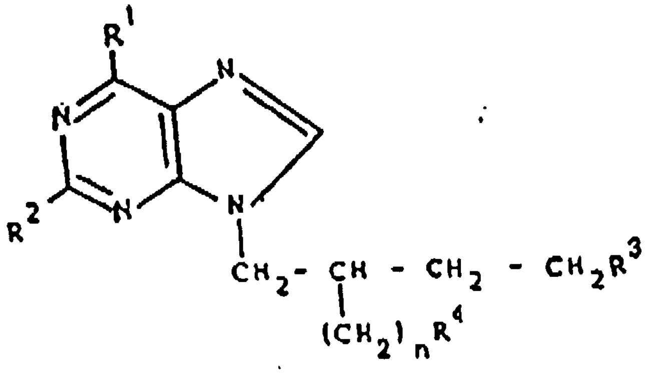

I

dans laquelle $R^1$, $R^2$, $R^3$, $R^4$, $R^5$ et n sont tels que définis dans la revendication 1, par

A. condensation d'une chaîne latérale acyclique

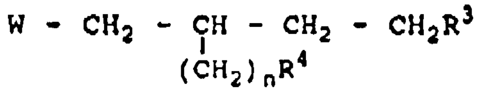

dans laquelle W est un groupe terminal mobile, en position N-9 d'un dérivé de la purine

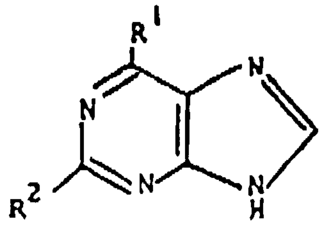

B. fermeture du cycle imidazole d'un dérivé de pyrimidine de formule

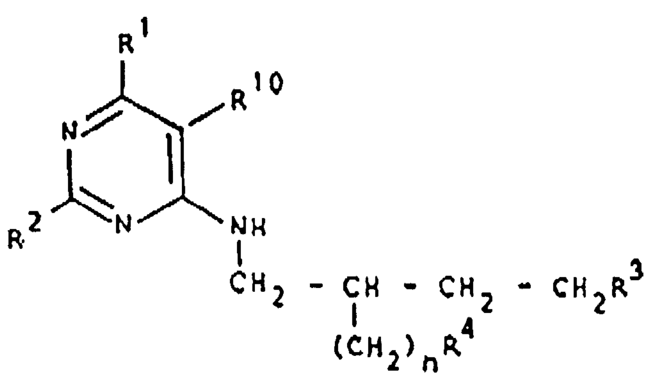

dans laquelle $R^{10}$ est un groupe amino ou un dérivé amino;

C. fermeture du cycle imidazole d'un noyau furazano-[3,4-d]pyrimidine de formule

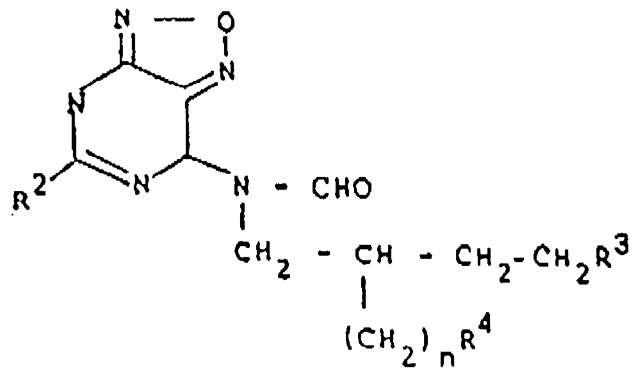

et coupure réductrice du noyau en un composé de formule I, dans laquelle $R^1$ est un groupe amino; ou
D. fermeture du cycle pyrimidine d'un dérivé imidazole de formule

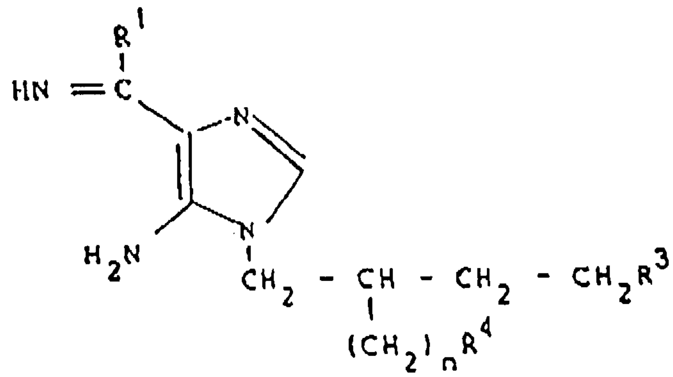

dans lesquels procédés, $R^1$ à $R^4$ et n sont tels que définis dans la revendication 1 et peuvent être éventuellement protégés par des groupes protecteurs convenables, de façon à fournir un mélange d'isomères optiques ou un isomère optique unique, le mélange racémique obtenu pouvant être éventuellement séparé en isomères optiques.
11. Utilisation d'un composé de formule I :

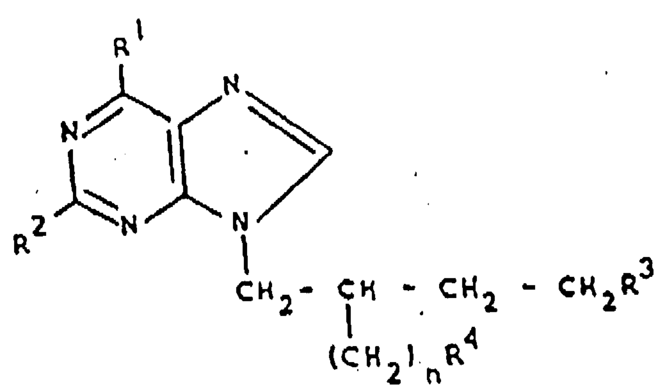

I

dans laquelle:
$R^1$ est un atome d'hydrogène, un groupe hydroxy, mercapto ou amino;
$R^2$ est un atome d'hydrogène, de fluor, de chlore, un groupe hydroxy ou amino;
$R^3$ et $R^4$ sont indépendamment choisis parmi

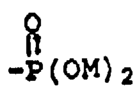, 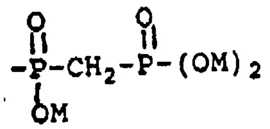, amino, hydroxy,

amino, hydroxy,

ou un résidu éther de ceux-ci défini en tant que $OR^5$, dans lequel $R^5$ est un groupe alkyle en $C_{1-6}$, arylalkyle éventuellement substitué avec un ou plusieurs groupes alcoxy, amino, nitrile ou sulfonamido ou un ou plusieurs atomes d'halogènes, ou un résidu ester de ceux-ci dérivé d'un acide carboxylique $R^6COOH$, d'un acide carbonique $R^7OCOOH$, d'un ester double d'un acide carbonique $R^7CO_2CH(R^8)OCO_2H$, d'un acide sulfonique $R^7SO_2OH$, d'un acide carbamique $R^7NHCOOH$, ou d'un acide phosphorique, dans lesquels $R^6$ est un atome d'hydrogène, un groupe alkyle en $C_{1-17}$, alcoxyalkyle, arylalkyle ou aryle, $R^7$ est un groupe alkyle en $C_{1-17}$, arylalkyle ou aryle, $R^8$ est un atome d'hydrogène ou un groupe alkyle en $C_{1-13}$ et ces groupes aryles et arylalkyles peuvent éventuellement être substitués avec un ou plusieurs groupes alkyle, alcoxy, amino, nitrile, ou sulfonamide ou un ou plusieurs atomes d'halogène,

ou $R^3$ forme avec $R^4$ un groupe

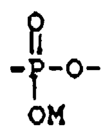

dans lequel

M est un atome d'hydrogène ou un contre-ion acceptable du point de vue pharmaceutique;

et n est 1 ou 2; et des sels de celui-ci acceptables du point de vue pharmaceutique pour la fabrication d'un médicament pour un traitement thérapeutique et/ou prophylactique du syndrome immunodéficitaire acquis et d'infections provoquées par des virus requérant une reverse transcriptase pour leur réplication.

12. Utilisation d'un composé de formule suivant la revendication 11 sous la forme d'un isomère optique.

13. Utilisation suivant la revendication 11 ou la revendication 12 pour le traitement des infections provoquées par des virus HIV.

14. Utilisation suivant la revendication 11 ou la revendication 12 pour le traitement des infections provoquées par des virus de l'hépatite B.

**Revendications pour les Etats contractants suivants : ES, GR**

1. Procédé pour la préparation d'un composé de formule I

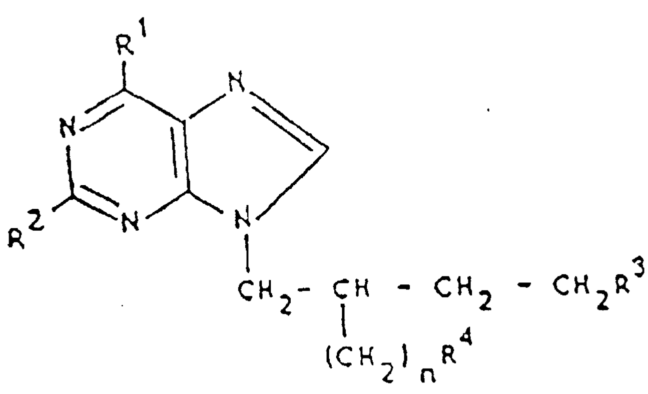

I

dans laquelle :

$R^1$ est un composé d'hydrogène, un groupe hydroxy, mercapto ou amino;

$R^2$ est un atome d'hydrogène, de fluor, de chlore, un groupe hydroxy ou amino;

$R^3$ et $R^4$ sont indépendamment choisis parmi

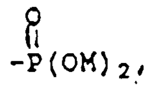, 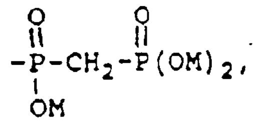,

amino, hydroxy,
ou un résidu éther de ceux-ci défini en tant que $OR^5$, dans lequel $R^5$ est un groupe alkyle en $C_{1-6}$, arylalkyle éventuellement substitué avec un ou plusieurs groupes alcoxy, amino, nitrile ou sulfonamido ou un ou plusieurs atomes d'halogène, ou un résidu ester de ceux-ci dérivé d'un acide carboxylique $R^6COOH$, d'un acide carbonique $R^7OCOOH$, d'un ester double d'un acide carbonique $R^7CO_2CH(R^8)OCO_2H$, d'un acide sulfonique $R^7SO_2OH$, d'un acide carbamique $R^7NHCOOH$ d'un acide phosphorique, dans lesquels $R^6$ est un atome d'hydrogène, un groupe alkyle en $C_{1-17}$, alcoxyalkyle, arylalkyle ou aryle, $R^7$ est un groupe alkyle en $C_{1-17}$, arylalkyle ou aryle, $R^8$ est un atome d'hydrogène ou un groupe alkyle en $C_{1-13}$ et ces groupes aryles et arylalkyles peuvent éventuellement être substitués avec un ou plusieurs groupes alkyle, alcoxy, amino, nitrile ou sulfonamide ou un ou plusieurs atomes d'halogène,
ou $R^3$ forme avec $R^4$ un groupe

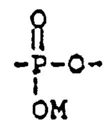

dans lequel
M est un atome d'hydrogène ou un contre-ion acceptable du point de vue pharmaceutique;
et n est 1 ou 2; à condition que, lorsque $R^2$ est un groupe amino et que $R^3$ et $R^4$ sont un groupe hydroxy, $R^1$ ne soit pas un groupe hydroxy et que de plus, lorsque n = 1, $R^1$ ne soit pas un atome d'hydrogène, et des sels de celui-ci acceptables du point de vue pharmaceutique, par

A. condensation d'une chaîne latérale acyclique

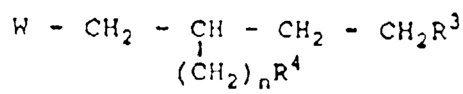

dans laquelle W est un groupe terminal mobile, en position N-9 d'un dérivé de la purine

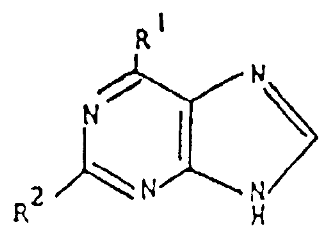

B. fermeture du cycle imidazole d'un dérivé de pyrimidine de formule

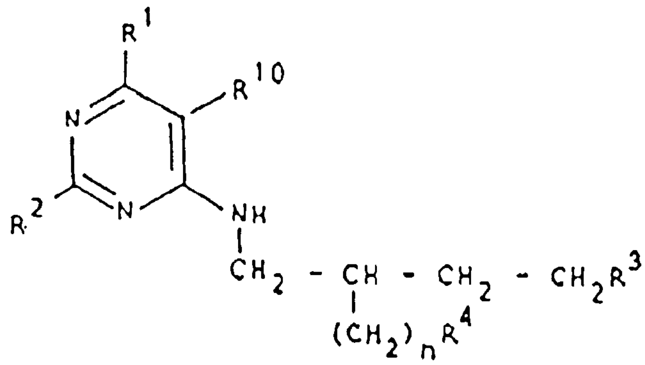

dans laquelle $R^{10}$ est un groupe amino ou un dérivé amino;

C. fermeture du cycle imidazole d'un noyau furazano-[3,4-d] pyrimidine de formule

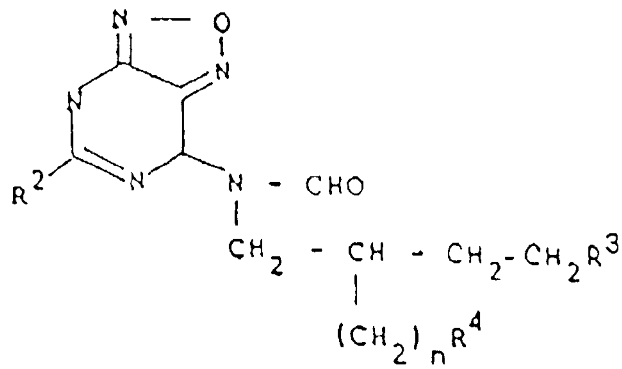

et coupure réductrice du noyau furazane en un composé de formule I, dans laquelle $R^1$ est un groupe amino; ou

D. fermeture du cycle pyrimidine d'un dérivé imidazole de formule

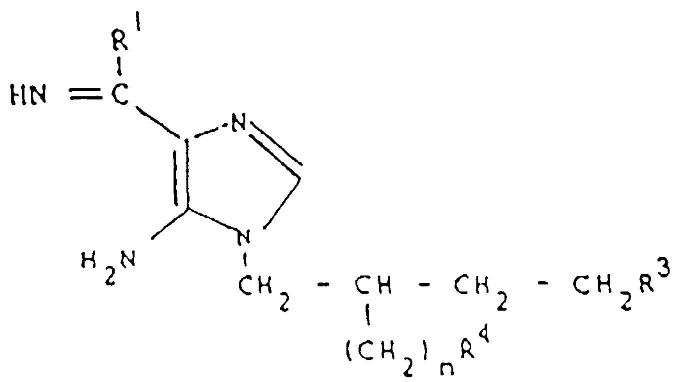

dans lesquels procédés, $R^1$ à $R^4$ et n sont tels que définis dans la revendication 1 et peuvent être éventuellement protégés par des groupes protecteurs convenables, de façon à fournir un mélange d'isomères optiques ou un isomère optique unique, le mélange racémique obtenu pouvant être éventuellement séparé en isomères optiques.

2. Procédé suivant la revendication 1 pour la préparation d'un composé de formule I sous la forme d'un isomère optique.

3. Procédé suivant la revendication 1 ou la revendication 2 pour la préparation d'un composé de formule I, dans lequel $R^3$ et $R^4$ sont tous les deux un groupe hydroxy.

4. Procédé suivant la revendication 1 ou la revendication 2 pour la préparation d'un composé de formule I, dans lequel $R^3$ est

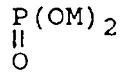

et $R^4$ est OH,
ou $R^3$ forme avec $R^4$ un groupe

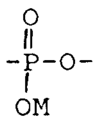

5. Procédé suivant l'une quelconque des revendications 1 à 4, pour utilisation en thérapie.

6. Procédé pour la préparation d'un composé de formule I suivant l'une quelconque des revendications 1 à 4, pour le traitement thérapeutique et/ou prophylactique des infections virales chez un hôte animal ou humain nécessitant un traitement.

7. Procédé pour la préparation d'un composé suivant la revendication 6 pour le traitement d'infections provoquées par les virus de l'herpès.

8. Procédé pour la préparation d'un composé suivant la revendication 6 pour le traitement d'infections provoquées par des virus nécessitant une reverse transcriptase pour leur réplication, comprenant les virus d'immunodéficience humaine et le virus de l'hépatite B.

9. Procédé pour la préparation d'une composition pharmaceutique comprenant la combinaison, en tant que composant actif, d'un composé suivant l'une quelconque des revendications 1 à 4 et d'un support acceptable du point de vue pharmaceutique.

10. Utilisation d'un composé de formule I:

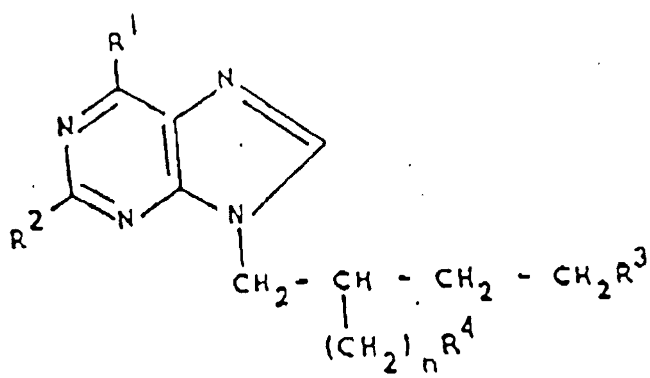

I

dans laquelle:
$R^1$ est un atome d'hydrogène, un groupe hydroxy, mercapto ou amino:
$R^2$ est un atome d'hydrogène, de fluor, de chlore, un groupe hydroxy ou amino;
$R^3$ et $R^4$ sont indépendamment choisis parmi

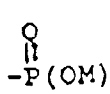,
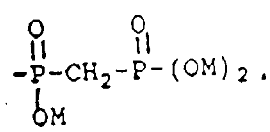

amino, hydroxy,
ou un résidu éther de ceux-ci défini en tant que $OR^5$, dans lequel $R^5$ est un groupe alkyle en $C_{1-6}$, arylalkyle éventuellement substitué avec un ou plusieurs groupes alcoxy, amino, nitrile ou sulfonamido ou un ou plusieurs atomes d'halogène, ou un résidu ester de ceux-ci dérivé d'un acide carboxylique $R^8COOH$, d'un acide carbonique $R^7OCOOH$, d'un ester double d'un acide carbonique $R^7CO_2CH(R^8)OCO_2H$, d'un acide sulfonique $R^7SO_2OH$, d'un acide carbamique $R^7NHCOOH$ ou d'un acide phosphorique, dans lesquels $R^6$ est un atome

d'hydrogène, un groupe alkyle en $C_{1-17}$, alcoxyalkyle, arylalkyle ou aryle, $R^7$ est un groupe alkyle en $C_{1-17}$, arylalkyle ou aryle, $R^8$ est un atome d'hydrogène ou un groupe alkyle en $C_{1-13}$ et ces groupes aryles et arylalkyles peuvent éventuellement être substitués avec un ou plusieurs groupes alkyle, alcoxy, amino, nitrile ou sulfonamide ou un ou plusieurs atomes d'halogène,
ou $R^3$ forme avec $R^4$ un groupe

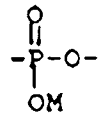

dans lequel
M est un atome d'hydrogène ou un contre-ion acceptable du point de vue pharmaceutique;
et n est 1 ou 2; et des sels de celui-ci acceptables du point de vue pharmaceutique pour la fabrication d'un médicament pour un traitement thrapeutique et/ou prophylactique du syndrome immunodéficitaire acquis et d'infections provoquées par des virus requérant une reverse transcriptase pour leur réplication.

11. Utilisation d'un composé de formule suivant la revendication 10 sous la forme d'un isomère optique.

12. Utilisation suivant la revendication 10 ou la revendication 11 pour le traitement des infections provoquées par des virus HIV.

13. Utilisation suivant la revendication 10 ou la revendication 11 pour le traitement des infections provoquées par des virus de l'hépatite B.